# FASCICULE DE BREVET EUROPEEN

(11) **EP 1 440 977 B1**
(45) Date de publication et mention de la délivrance du brevet: **26.07.2006**
(21) Numéro de dépôt: 04300039.7
(22) Date de dépôt: 22.01.2004
(51) Int. Cl.: C07K 5/08, A61K 8/64

(54) **Procédé de synthèse amélioré de dérivés diamides du tripeptide KPV**
Verbessertes Verfahren zur Herstellung von diamidischen Derivaten vom Tripeptid KPV
Improved process for the preparation of diamidic derivatives of the tripeptid KPV

(30) Priorité: 24.01.2003 FR 0300808
(43) Date de publication de la demande: 28.07.2004
(73) Titulaire: L'ORÉAL, 75008 Paris (FR)
(72) Inventeur: Genard, Sylvie, 94130 Nogent sur Marne (FR)
(74) Mandataire: Catherine, Alain

(56) Documents cités:
- WO-A-88/00833
- WO-A-02/069884
- DATABASE CA [Online] CHEMICAL ABSTRACTS SERVICE, COLUMBUS, OHIO, US; 1975, EBERLE, ALEX ET AL: "Hormone-receptor interactions. Syntheses of.alpha.-melanotropin and information-carrying sequences using alkali-labile protecting groups" XP002263994 extrait de STN Database accession no. 84:59984 CA & HELVETICA CHIMICA ACTA ( 1975 ), 58(7), 2106-29, 1975,

## Description

La présente invention se rapporte à un procédé amélioré de synthèse de dérivés diamides du tripeptide Lysine-Proline-Valine (JPV).

### ART ANTERIEUR

En synthèse peptique, on distingue deux grandes voies de synthèse qui sont la synthèse en phase solide et la synthèse en solution.

La synthèse en phase solide présente l'avantage d'une grande souplesse et d'une automatisation déjà existante. Les produits sont en général synthétisés à des échelles laboratoire allant dans la majorité des cas de quelques milligrammes à une centaine de milligrammes. Son principe repose sur celui de la chimie sur support avec ses avantages connus en particulier en terme de purification ce qui permet la mise en oeuvre d'excès de réactifs pour favoriser le rendement. Cette voie est en général utilisée pour la synthèse d'un grand nombre de composés dans un but de criblage de leur activité.

La synthèse en solution présente, quant à elle, l'avantage de pouvoir préparer de grandes quantités de produit en un seul lot. Toutes les réactions sont réalisées en solution sans utilisation de réactif supporté sur une résine comme dans le cas précédent. C'est, en synthèse peptidique, la méthode de choix pour une synthèse industrielle.

On peut également citer les réactions d'hémisynthèse à partir de peptides naturels. Elles consistent en l'hydrolyse ménagée (le plus souvent par voie enzymatique) de peptides naturels conduisant à un mélange de fragments de nature peptidique qui sont séparés puis éventuellement dérivés pour obtenir les composés souhaités.

### Synthèse en solution

A la connaissance du demandeur, une seule publication décrit la synthèse en solution de composés diamides du tripeptide KPV, le seul composé exemplifié étant Ac-Lys-Pro-Val-NH₂ (Eberle et al., 1975).

Dans cette synthèse, la chaîne latérale de la lysine est protégée par un groupe protecteur MSOC introduit sur Boc-Lys-OH pour former Boc-Lys(MSOC)-OH. La lysine N(α),N(ε) diprotégée est couplée avec Boc-Pro-Val-NH₂, HCI par la méthode classique DCC/HOBt. Le réactif Boc-Pro-Val-NH₂, HCI est lui même obtenu en 2 étapes à partir de Boc-Pro-OH et de Boc-Val-NH₂. Le rendement global est de 33% calculé par rapport à Boc-Lys(MSOC)-OH. Le schéma de synthèse global est illustré dans le schéma (I) ci-dessous :

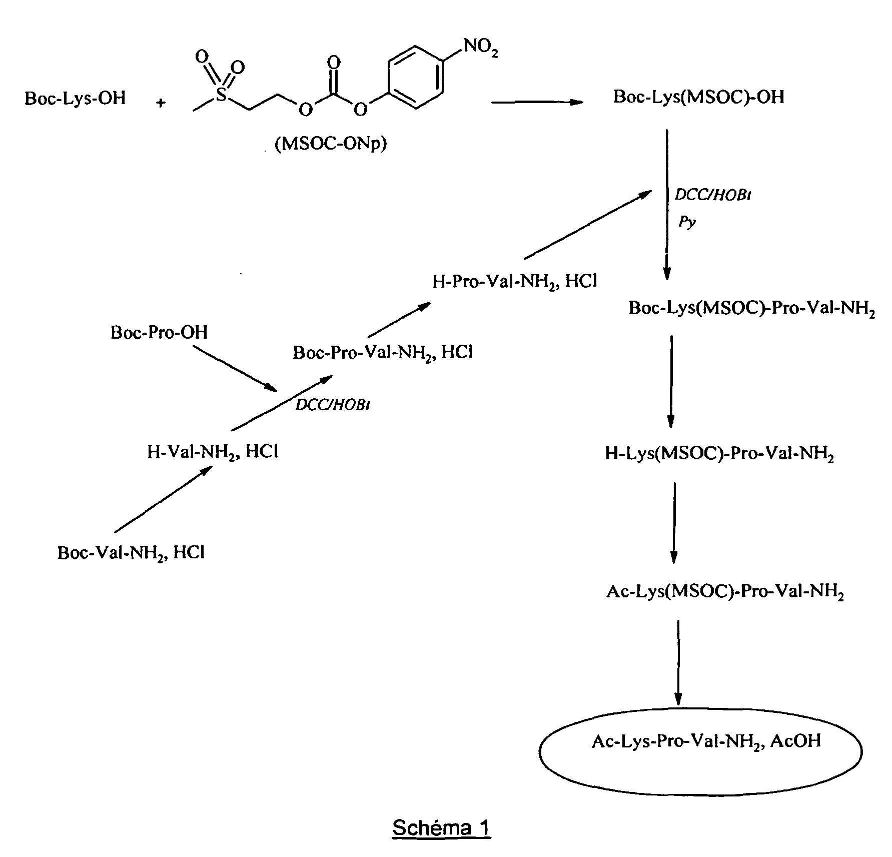

Une préparation sommaire et incomplète de ce même composé Ac-Lys-Pro-Val-NH₂ (sel non spécifié) et de son homologue diacétylé Ac-Lys(Ac)-Pro-Val-NH₂ est citée dans la demande de brevet européen N° EP0317573 (Lipton).

Dans d'autres documents, tels que la demande internationale n°WO0056353 (Lipton), le composé Ac-Lys-Pro-Val-NH₂ est cité mais seule une indication du mode de synthèse est donnée. Il est précisé que « les peptides ont été préparés par synthèse peptidique en phase solide et purifiés par chromatographie liquide haute performance en phase inverse ».

### En Phase solide:

En phase solide, la synthèse de Ac-Lys-Pro-Val-NH2 est décrite en série L avec un rendement global de 56.1%.(Sawyer, 1981)

Toujours en phase solide, l'article de Staples et al. (1985) décrit une synthèse peptidique permettant l'obtention du composé Ac-Lys-Pro-Val-NH₂ mais avec une incohérence entre la structure et les analyses d'acides aminés indiquant la présence de Glycine. La méthodologie en phase solide utilisée par Sawyer dans sa thèse (Sawyer, 1981) est généralisée à la synthèse de peptides Ac-Peptide-NH₂ (Sawyer, 1982) sans que le tripeptide Ac-Lys-Pro-Val-NH₂ soit exemplifié ni cité. Dans cette méthodologie (Sawyer, 1981 ; 1982), la résine est une résine de type p-méthylbenzhydrylamine qui permet l'obtention directe avec de bons rendements de la forme carboxamide (CO-NH₂) après clivage du peptide de la résine par l'acide fluorhydrique HF. Les acides aminés sont introduits sous leur forme N-protégée par un Boc et couplés par la technique classique DCC/HOBt. Dans le cas de la lysine, celle-ci est introduite sous forme de N(α)Boc, N(α)-2,4-Cl2-Z-Lys-OH. Lorsque le peptide est synthétisé, la protection de l'amine N(α) du dernier acide aminé couplé est déprotégée et l'acide aminé terminal est acétylé par un excès de N-acétylimidazole avant clivage de la résine. Par cette méthodologie, le tripeptide Ac-Lys-Pro-Val-NH2 est synthétisé avec un rendement global de 56,1% (Sawyer, 1981), selon le schéma 2 ci-dessous.:

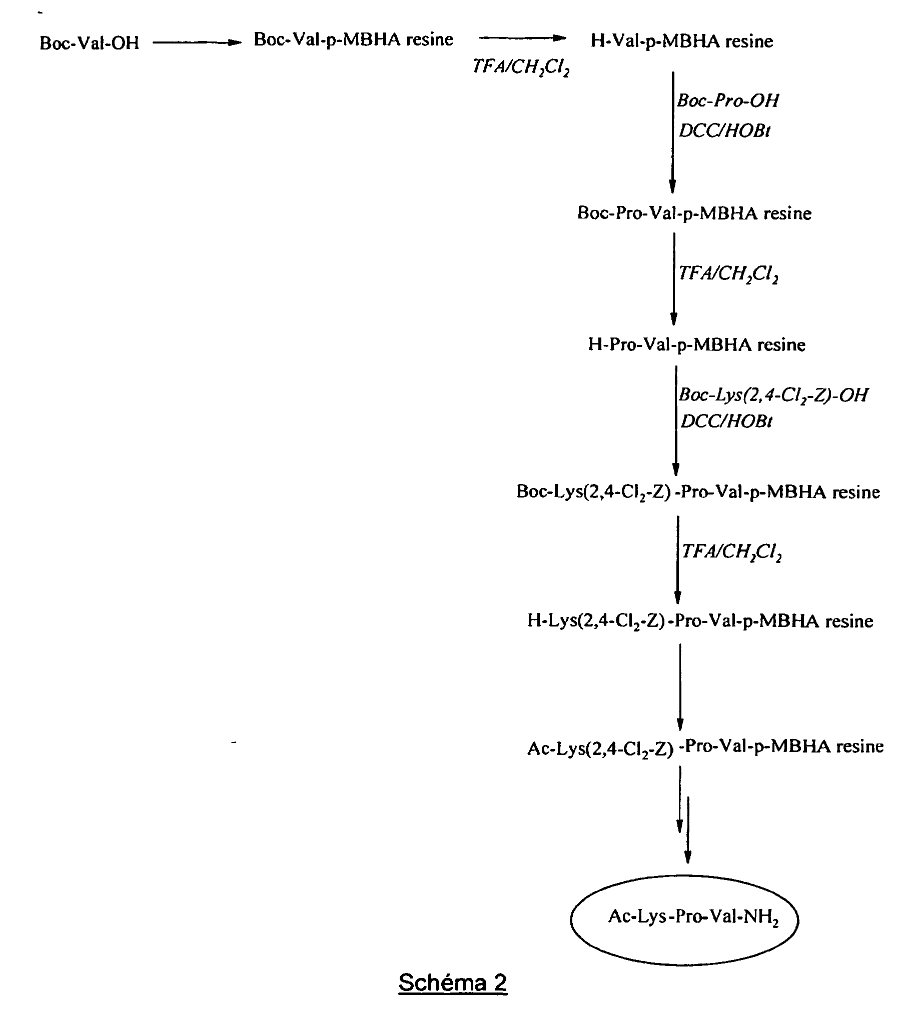

### Principe général des synthèses décrites dans l'état de la technique:

Dans le schéma 1, l'intermédiaire clé permettant l'obtention de diamides du tripeptide KPV est Boc-Lys(MSOC)-Pro-Val-NH₂, les deux groupes protecteurs étant libérés dans des conditions opératoires différentes. En libérant spécifiquement le groupe protecteur N(α) de la lysine (Boc) , la fonction amine libre est amidifiée puis la fonction amine N(ε) de la lysine est à son tour libérée.

Plus généralement, la synthèse de dérivés de type (I) peut être envisagée à partir d'intermédiaires de synthèse du tripeptide H-Lys-Pro-Val-OH (ou de dérivés) dès lors que les deux fonctions amines N(α) et N(ε) de la lysine sont protégées par des groupes protecteurs labiles dans des conditions opératoires différentes.

Les dérivés diprotégés du tripeptide sont décrits dans l'état de la technique.

Ainsi, le brevet américain US 5580855 (Ferreira) décrit la synthèse en phase solide du tripeptide H-Lys-Pro-Val-OH en utilisant le FMOC comme groupe protecteur de la fonction amine en α et comme phase solide un polymère polyméthacrylamide synthétisé à partir des trois monomères différents. L'introduction des acides aminés se fait via leur activation sous forme d'anhydride symétrique. Dans le cas de la lysine, la fonction amine N(ε) est protégée par un Boc. A l'issue de la synthèse, le tripeptide est libéré du support solide par utilisation d'acide trifluoroacétique (qui déprotège aussi les groupes Boc), le FMOC étant préalablement libéré par la pipéridine à 20% dans le DMF.

La préparation de l'intermédiaire H-Lys(Boc)-Pro-Val-NH₂ (ou de ses sels) est décrite notamment par Suli-Vargha et al. (1987), Suli-Vargha et al. (1984), Schwyzer et al. (1963) ainsi que dans la demande de brevet européen publiée sous le n° EP 78167.

Parmi les autres composés intermédiaires protégés, on peut citer par exemple H-Lys(formyl)-Pro-Val-NH2, HCI préparé à partir de Z-Lys(formyl)-Pro-Val-NH₂ (Suli-Vargha et al., 1987), ou H-Lys(Z)-Pro-Val-NH₂ (ou de ses sels) (Yasutake and Powers, 1981), H-Lys(Tosyl)-Pro-Val-NH₂ (Hofmann et al., 1960).

Comme exposé précédemment, les procédés de synthèse de dérivés diamides du tripeptide KPV de l'état de la technique possèdent des inconvénients :
(a) les procédés de synthèse en phase solide permettent l'obtention de dérivés diamides du peptide KPV avec un degré de pureté satisfaisant mais ne sont adaptés qu'à la production de faibles quantités de produit final, de quelques milligrammes à quelques centaines de milligrammes, incompatibles avec des besoins industriels ;
(b) les procédés de synthèse en solution permettent la préparation de grandes quantités du produit final, mais avec un rendement insuffisant, de l'ordre de 33%. Ces procédés de synthèse en solution nécessitent des étapes ultérieures de purification du produit final, longues et coûteuses, et entraînent une perte quantitative significative du produit, lorsque l'on passe de la forme non purifiée à la forme purifiée.

Il existe donc un besoin dans l'état de la technique pour un procédé de synthèse de dérivés diamides du tripeptide KPV pour obtenir directement de grandes quantités du produit final, à haut rendement, et sans nécessiter d'étape spécifique de purification.

### SOMMAIRE DE L'INVENTION

Un tel procédé de synthèse d'un dérivé diamide du tripeptide KPV est fourni selon l'invention. Le procédé de synthèse selon l'invention peut être réalisé avec n'importe lequel des stéréoisomères de chacun des résidus d'acide aminé Lysine (K), Proline (P) ou Valine (V).

L'invention a pour objet un procédé de synthèse en solution d'un dérivé diamide du tripeptide KPV de formule (I), ou encore d'un sel d'un dérivé de formule (I) suivante : 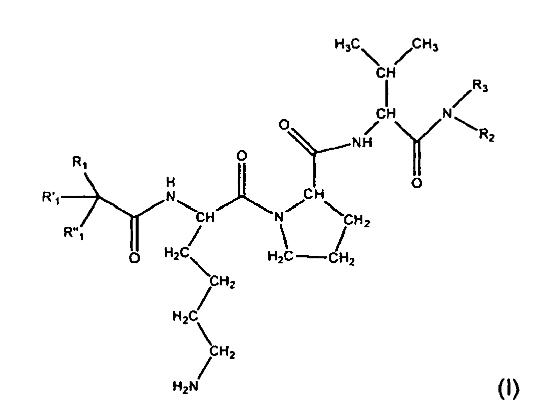 indépendamment de la stéréochimie des acides aminés mis en oeuvre dans laquelle :
a) R₁, R'₁ et R"₁ représentent, indépendamment l'un de l'autre, un atome d'hydrogène ou
   - alkyle C₁-C₂₂, linéaire ou ramifié, éventuellement interrompu par un hétéroatome tel que O ou N ou S ou Si
   - cycloalkyle C₄-C₁₀
   - polyfluoroalkyle ou perfluoroalkyle C₁-C₂₂, linéaire ou ramifié
   - aryle éventuellement substitué par un ou plusieurs atomes d'halogène tels que CI, F , Br ou I ou, un ou plusieurs groupes alkyles C₁-C₄ linéaires ou ramifiés.
   - aralkyle
   - ou R₁ et R'₁ peuvent former avec C(R"₁) un cycle saturé de 3 à 7 atomes, éventuellement substitué par un ou plusieurs radicaux alkyles C₁-C₄ linéaires ou ramifiés et/ou renfermant éventuellement un hétéroatome tel que O, S ou N,
   à condition que le groupe R₁(R'₁)(R"₁)CO ne représente pas un résidu acide aminé ou un reste peptidique;
b) R₂ et R₃ représentent, indépendamment l'un de l'autre un atome d'hydrogène, ou représentent
   - alkyle C₁-C₂₄, linéaire ou ramifié, éventuellement interrompu par un hétéroatome tel que O ou N ou S ou Si
   - cycloalkyle C₄-C₁₀
   - polyfluoroalkyle ou perfluoroalkyle C₁-C₂₂, linéaire ou ramifié
   - aryle éventuellement substitué par un ou plusieurs atomes d'halogène tels que CI, F , Br ou I ou, un ou plusieurs groupes alkyles C₁-C₄ linéaires ou ramifiés.
   - aralkyle
   - ou R₂ et R₃ peuvent former avec l'atome d'azote un cycle saturé de 5 ou 6 atomes éventuellement substitué par un ou plusieurs radicaux alkyle C₁-C₄ linéaires ou ramifiés, cycle saturé renfermant éventuellement un hétéroatome tel que O, S ou encore un atome d'azote supplémentaire ,
à condition que le groupe N(R₂) (R₃) ne représente pas un aminoacide ou un reste peptidique ;
ledit procédé étant caractérisé en ce qu'il comprend les étapes suivantes :
a) réaction d'un résidu de lysine diprotégé de formule (II) suivante : 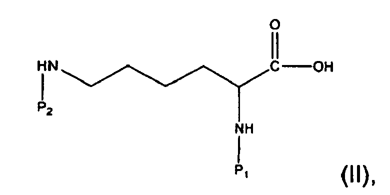 éventuellement salifié par une base minérale ou organique,
   dans laquelle P₁ et P₂, différents l'un de l'autre, représentent chacun indépendamment l'un de l'autre un groupe protecteur,
   avec un résidu de Proline de formule (III) suivante : 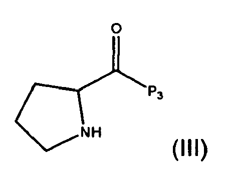 éventuellement salifié par un acide minéral ou organique,
   dans laquelle P₃ représente un groupe protecteur différent de l'un quelconque des groupes protecteurs P₁ et P₂, ou dans laquelle P₃ représente un groupe hydroxyle, en présence d'un réactif d'activation ou d'un réactif de couplage dans un solvant, afin d'obtenir le composé de formule (IV) suivant : 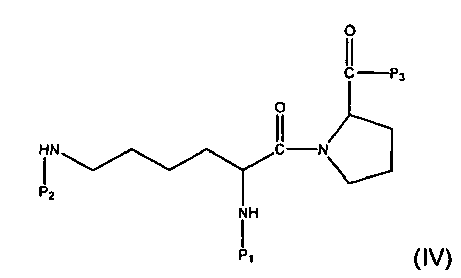 dans laquelle P₁, P₂ et P₃ ont les significations ci-dessus.
b)
   1) couplage, sur la fonction C-terminale du résidu Proline du composé de formule (IV) dans lequel P₃ représente OH, d'un composé de valine de formule (V) suivante : 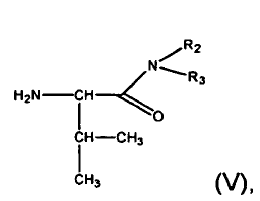 dans laquelle R₂ et R₃ ont la même signification que ci-dessus, élimination du groupe protecteur P₁,
   2) amidification du groupe NH₂(α) en position N-terminale du résidu lysine par un composé de formule (VI-A) ou (VI-B) suivante : 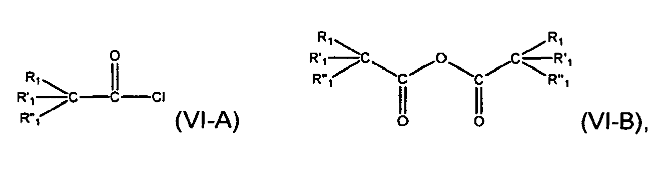 afin d'obtenir le composé de formule (XII) suivant : 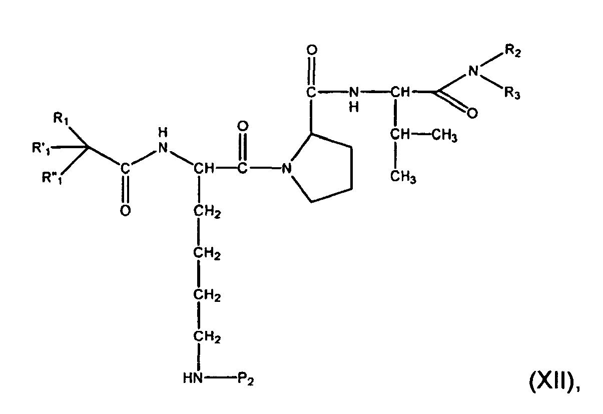
   dans laquelle P₂, R₁, R'₁, R"₁, R₂ et R₃ ont la même signification que ci-dessus ;
   l'ordre des étapes 1) et 2) étant indifférent ;
c) Eliminer le groupe protecteur P₂ du composé de formule (XII) afin d'obtenir le composé de formule (I), éventuellement sous la forme d'un sel minéral ou organique.

Dans la formule (I), les substituants R₁, R'₁ et R"₁ sont identiques ou différents. Ils peuvent prendre les valeurs:
- hydrogène
- alkyle C₁-C₂₂, linéaire ou ramifié, éventuellement interrompu par un hétéroatome tel que O ou N ou S ou Si
- cycloalkyle C₄-C₁₀
- polyfluoroalkyle ou perfluoroalkyle C₁-C₂₂, linéaire ou ramifié
- aryle éventuellement substitué par un ou plusieurs atomes d'halogène tels que CI, F , Br ou I ou, un ou plusieurs groupes alkyles C₁-C₄ linéaires ou ramifiés.
- aralkyle
- R₁ et R'₁ peuvent former avec C(R"₁) un cycle saturé de 3 à 7 atomes, éventuellement substitué par un ou plusieurs radicaux alkyle C₁-C₄ linéaires ou ramifiés et/ou renfermant éventuellement un hétéroatome tel que O, S ou N.

En revanche, le groupe R₁(R'₁)(R"₁)CO ne peut représenter un reste acide aminé ou une chaîne peptidique.

Dans la formule (I), les substituants R₂ et R₃ sont identiques ou différents. Ils peuvent prendre les valeurs:
- hydrogène
- alkyle C₁-C₂₄, linéaire ou ramifié, éventuellement interrompu par un hétéroatome tel que O ou N ou S ou Si
- cycloalkyle C₄-C₁₀
- polyfluoroalkyle ou perfluoroalkyle C₁-C₂₂, linéaire ou ramifié
- aryle éventuellement substitué par un ou plusieurs atomes d'halogène tels que CI, F , Br ou I ou, un ou plusieurs groupes alkyles C₁-C₄ linéaires ou ramifiés.
- aralkyle
- R₂ et R₃ peuvent former avec l'atome d'azote un cycle saturé de 5 ou 6 atomes, éventuellement substitué par un ou plusieurs radicaux alkyles C₁-C₄ linéaires ou ramifiés, cycle saturé renfermant éventuellement un hétéroatome tel que O, S ou encore un atome d'azote supplémentaire.

En revanche le groupe N(R₂)(R₃) ne peut constituer un amino-acide ou une chaîne peptidique.

Par « alkyle », on entend un groupe hydrocarboné aliphatique linéaire ou ramifié, éventuellement interrompu par un hétéroatome, l'alkyle pouvant être non substitué ou substitué sur les atomes de carbone par un ou plusieurs substituants identiques ou différents, les substituants étant choisis parmi : aryle, hydroxy, alcoxy, aryloxy, alkyloxy, aralkyloxy. Par alkyle « ramifié », on entend un alkyle inférieur tel que méthyle, éthyle ou propyle qui est lié à une chaîne linéaire alkyle. Les groupes alkyles préférés comprennent les groupes « alkyle inférieur » ayant de 1 à 8 atomes de carbone. Des exemples de groupes alkyles sont methyle, éthyle, *i*-propyle, *t*-butyle, heptyle, decyle ou cyclohexylmethyle.

Par « cycloalkyle », on entend un cycle non aromatique comprenant de 4 à 10 atomes de carbone, l'alkyle cyclique pouvant être partiellement insaturé. Des cycloalkyles préférés incluent cyclopentyle, cyclohexyle, cycloheptyle, adamantyle, octahydronaphthyle et perhydronaphthyle.

Par « aryle », on entend un radical carbocyclique aromatique contenant de 5 à 10 atomes de carbone. Des groupes aryles incluent le phényle ou le naphtyle non substitués ou le phényle ou le naphtyle substitués par un ou plusieurs substituants qui peuvent être identiques ou différentes, incluant les substituants alkyle, aryle, aralkyle, hydroxy , alcoxy, aryloxy, aralcoxy, carboxy, aroyl, halo, nitro, trihalométhyle, cyano, alcoxycarbonyle, aryloxycarbonyle, aralcoxycarbonyle, carbamoyle, alkylcarbamoyle, dialkylcarbamoyle.

Par « alcoxy », on entend un groupe alkyle-O- dans le quel le groupe alkyle est tel que défini précédemment. Les groupes alcoxy incluent méthoxy, éthoxy, *n*-propoxy, *i*-propoxy, *n*-butoxy et heptoxy.

Par « aryloxy », on entend un groupe aryle-O dans lequel le groupe aryle est tel que défini précédemment. Les groupes aryloxy incluent le phénoxy et le naphthoxy.

Par « aralkyle », on entend un groupe alkyle substitué par un ou plusieurs groupes aryles.

Par « aralkyloxy », on entend un groupe aralkyle-O- dans lequel le groupe aralkyle est tel que défini précédemment. Les groupes aralkyloxy incluent le benzyloxy.

Par « alcoxycarbonyle », on entend un groupe -O-CO-, tel que méthoxy- et ethoxy-carbonyle.

Par « aryloxycarbonyle », on entend un groupe aryle-O-CO-, tel que phenoxy- et naphthoxy-carbonyle.

Par « halo », on entend un atome de fluor, de chlore ou de brome.

Le procédé selon l'invention permet en particulier l'obtention de ces dérivés sous forme salifiée. Les sels des composés conformes à l'invention sont choisis parmi les sels d'un acide minéral ou organique comme par exemple, les chlorhydrates, bromhydrates, sulphate, acétate, citrate, tartrate, lactate, phosphate, hydrogénophosphate, propionate ou succinate.

Le procédé selon l'invention présente entre outre l'avantage d'être applicable à l'échelle industrielle, pour la production de lots de dérivés diamides du tripeptide KPV de plusieurs kilogrammes et ce, quelle que soit la stéréochimie des acides aminés mis en oeuvre.

L'invention concerne également les dérivés diamides du tripeptide KPV de formule (I) définis ci-dessus avec la condition supplémentaire que R₁, R'₁, R"₁, R₂ et R₃ ne représentent pas simultanément un atome d'hydrogène (formule IA).

Ces dérivés peuvent être sous forme de sels, en particulier ceux cités précédemment.

### DESCRIPTION DES FIGURES

La **figure 1A** illustre un mode de réalisation du procédé de couplage entre le résidu lysine et le résidu proline, dans lequel la fonction carboxyle du résidu proline est protégée par un groupe protecteur P₃.
La **Figure 1B** illustre un mode de réalisation du procédé de couplage entre le résidu lysine et le résidu proline, dans lequel la fonction carboxyle du résidu proline est libre (P₃=OH).
La **Figure 2** illustre le premier mode de réalisation préféré de l'étape b) du procédé selon l'invention.
La **Figure 3** illustre le second mode de réalisation préféré de l'étape b) du procédé selon l'invention.
La **Figure 4** illustre le troisième mode de réalisation préféré de l'étape b) du procédé selon l'invention.
La **Figure 5** illustre l'étape c) du procédé selon l'invention.
La **Figure 6** représente le schéma réactionnel complet du meilleur mode de réalisation du procédé de synthèse de dérivés diamides du tripeptide KPV de l'invention.

### DESCRIPTION DETAILLEE DE L'INVENTION

II est montré selon l'invention qu'une combinaison particulière d'étapes de synthèse et l'utilisation d'une combinaison particulière de groupements protecteurs permet de préparer des dérivés diamides du tripeptide KPV, ou un sel de ces composés, dans un procédé de synthèse en solution avec un rendement final bien supérieur au rendement obtenu avec les procédés connus de l'état de la technique, et ne nécessitant aucune étape de purification finale, comme par exemple par chromatographie, notamment par chromatographie d'échange d'ions.

Il a été montré que le choix judicieux des groupes protecteurs, des réactifs utilisés et l'enchaînement des réactions permettaient de porter le rendement, pour une synthèse réalisée en solution, de 33% (Eberle et al., 1975) à plus de 70% dans le cas de Ac-Lys-Pro-Val-NH₂.

Les combinaisons réactifs/groupes protecteurs et l'enchaînement des étapes de réaction sont nouvelles pour la préparation des composés de formule (I).

De plus, le procédé de synthèse de l'invention est applicable à l'échelle industrielle. L'isolement du produit final est simple et ne nécessite aucune purification par chromatographie ou colonne échangeuse d'ions. Ces techniques lourdes ne sont mises en oeuvre à aucun stade de la synthèse. A l'issue de l'étape c) du procédé selon l'invention, le dérivé diamide du tripeptide KPV est obtenu directement avec une très grande pureté. En fin de synthèse, la pureté du produit isolé, cristallisé peut être supérieure à 95%, comme cela a été déterminé par chromatographie liquide haute performance (HPLC).

D'autre part, si le composé (I) doit être obtenu sous forme salifiée, cette salification est réalisée in situ lors de la dernière étape du procédé, consistant à éliminer le groupe protecteur P₂, et ne nécessite aucune réaction supplémentaire ou mise en oeuvre particulière.

L'invention a pour objet un procédé de synthèse en solution d'un dérivé diamide du tripeptide KPV de formule (I), ou encore d'un sel d'un dérivé de formule (I) suivante : 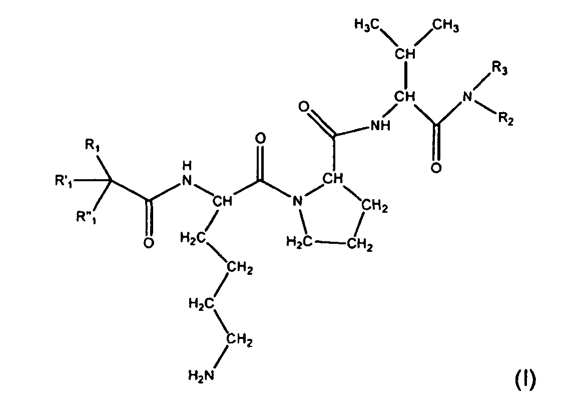 indépendamment de la stéréochimie des acides aminés mis en oeuvre, dans laquelle :
a) R₁, R'₁ et R"₁ représentent, indépendamment l'un de l'autre, un atome d'hydrogène ou
   - alkyle C₁-C₂₂, linéaire ou ramifié, éventuellement interrompu par un hétéroatome tel que O ou N ou S ou Si
   - cycloalkyle C₄ -C₁₀
   - polyfluoroalkyle ou perfluoroalkyle C₁-C₂₂, linéaire ou ramifié
   - aryle éventuellement substitué par un ou plusieurs atomes d'halogène tels que CI, F , Br ou I ou, un ou plusieurs groupes alkyles C₁-C₄ linéaires ou ramifiés.
   - aralkyle
   - ou R₁ et R'₁ peuvent former avec C(R"₁) un cycle saturé ou insaturé de 3 à 7 atomes, éventuellement substitué par un ou plusieurs radicaux alkyles C₁-C₄ linéaires ou ramifiés et/ou renfermant éventuellement un hétéroatome tel que O, S ou N,
   à condition que le groupe R₁(R'₁)(R"₁)CO ne représente pas un résidu d'acide aminé ou un reste peptidique;
b) R₂ et R₃ représentent, indépendamment l'un de l'autre un atome d'hydrogène, ou représentent
   - alkyle C₁-C₂₄, linéaire ou ramifié, éventuellement interrompu par un hétéroatome tel que O ou N ou S ou Si
   - cycloalkyle C₄ - C₁₀
   - polyfluoroalkyle ou perfluoroalkyle C₁-C₂₂, linéaire ou ramifié
   - aryle éventuellement substitué par un ou plusieurs atomes d'halogène tels que CI, F , Br ou I ou, un ou plusieurs groupes alkyles C₁-C₄ linéaires ou ramifiés.
   - aralkyle
   - ou R₂ et R₃ peuvent former avec l'atome d'azote un cycle saturé de 5 ou 6 atomes, éventuellement substitué par un ou plusieurs radicaux alkyles C₁-C₄ linéaires ou ramifiés, cycle saturé renfermant éventuellement un héréroatome tel que O, S ou encore un atome d'azote supplémentaire ,
      à condition que le groupe N(R₂)(R₃) ne représente pas un aminoacide ou un reste peptidique ;
ledit procédé étant caractérisé en ce qu'il comprend les étapes suivantes :
a) réaction d'un résidu de lysine diprotégé de formule (II) suivante : 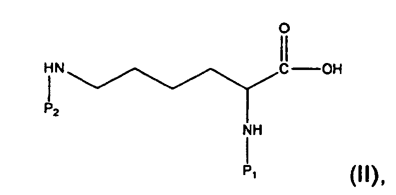 éventuellement salifié par une base minérale ou organique,
   dans laquelle P₁ et P₂, différents l'un de l'autre, représentent chacun indépendamment l'un de l'autre un groupe protecteur,
   avec un résidu de Proline de formule (III) suivante : 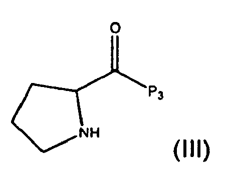 éventuellement salifié par un acide minéral ou organique,
   dans laquelle P₃ représente un groupe protecteur différent de l'un quelconque des groupes protecteurs P₁ et P₂, ou dans laquelle P₃ représente un groupe hydroxyle,
   en présence d'un réactif d'activation ou d'un réactif de couplage dans un solvant, afin d'obtenir le composé de formule (IV) suivant : 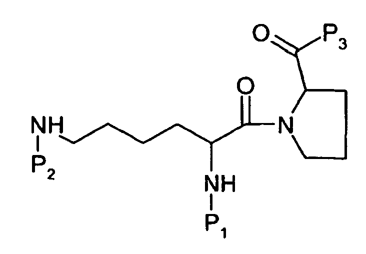 dans laquelle P₁, P₂ et P₃ ont les significations ci-dessus.
b)
   1) couplage, sur la fonction C-terminale du résidu Proline du composé de formule (IV) pour lequel P₃ représente OH, d'un composé de valine de formule (V) suivante : 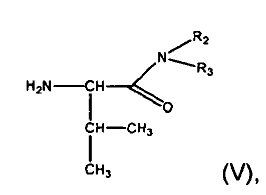 dans laquelle R₂ et R₃ ont la même signification que ci-dessus, élimination du groupe protecteur P₁ ;
   2) amidification du groupe NH₂(α) en position N-terminale du résidu lysine de formule (VI-A) ou (VI-B) suivante : 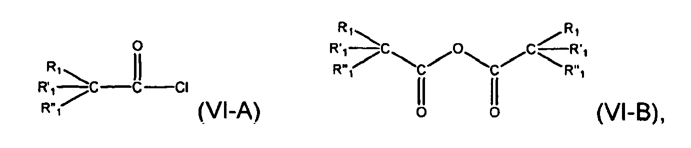 afin d'obtenir le composé de formule (XII) suivant : 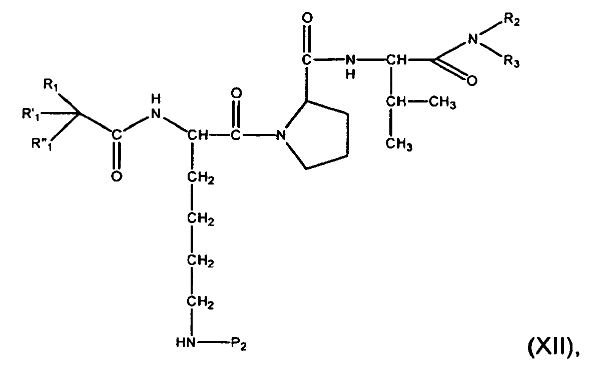
   dans laquelle P₂, R₁, R'₁, R"₁, R₂ et R₃ ont la même signification que ci-dessus ;
   l'ordre des étapes 1) et 2) étant indifférent ;
c) Eliminer le groupe protecteur P₂ du composé de formule (XII) afin d'obtenir le composé de formule (I), éventuellement sous la forme d'un sel minéral ou organique.

A l'étape c) l'élimination du groupe protecteur P₂ est réalisée par hydrogénolyse. Lorsque l'hydrogénolyse est réalisée avec un acide contenu dans le milieu réactionnel, ledit acide joue le rôle d'un agent salifiant de la fonction amine de la lysine ainsi libérée. Le sel final est de préférence choisi parmi les chlorhydrates, bromhydrates, sulfates, acétates, citrates, tartrates, lactates, phosphates, hydrogénophosphates, propionates et succinates.

Selon l'invention, le procédé tel que défini ci-dessus peut être réalisé avec n'importe lequel des stéréoisomères de chacun des résidus d'acides aminés Lysine, Proline ou Valine.

Préférentiellement, selon le procédé de l'invention, le sel est obtenu au cours de l'étape c) par introduction de l'acide correspondant.

Préférentiellement, l'acide utilisé est choisi parmi l'acide acétique, l'acide chlorhydrique, l'acide bromhydrique, l'acide sulfurique, l'acide citrique, l'acide tartrique, l'acide lactique, l'acide phosphorique, l'acide hydrogénophosphorique, l'acide propionique ou l'acide succinique.

De manière tout à fait préférée, l'acide est l'acide acétique ou chlorhydrique.

Selon un mode préféré de réalisation, le procédé de l'invention est en outre caractérisé en ce que les groupes protecteurs P₁ et P₂ représentent, indépendamment l'un de l'autre, Adoc (= 1-adamantyloxycarbonyle) BOC (=t-butyloxycarbonyle), 2-bromo-Z (= 2-bromo-benzyloxycarbonyle), ou 2-chloro-Z (=2-chloro-benzyloxycarbonyle) ou Fmoc (= 9-fluorenylméthoxycarbonyle) ou Formyle ou Nicotinoyle ou 4-nitro-Z (= 4-nitro-benzyloxycarbonyle) ou Tfa (= trifluoroacétyle) ou Tos (= p-toluènesulfonyle) ou Z(=benzyloxycarbonyle) ou adpoc (=1-(adamantyl)-1-méthyléthoxycarbonyle).

Pour préparer les composés de formule (I) en solution et/ou leurs sels, plusieurs voies de synthèse sont envisageables selon que la partie peptidique est construite de l'extrémité N-terminale vers l'extrémité C-terminale ou à l'inverse, de l'extrémité C-terminale vers l'extrémité N-terminale. Préférentiellement, la chaîne peptidique est construite de l'extrémité N-terminale vers l'extrémité C-terminale.

Le produit de départ du procédé de synthèse est la lysine diprotégée par 2 groupes protecteurs labiles dans des conditions opératoires différentes de structure : 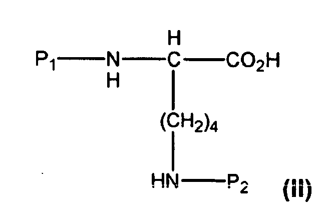 où P₁ et P₂ sont différents et peuvent désigner les groupes protecteurs suivants, à condition que P₁ soit différent de P₂ et que P₁ et P₂ soient labiles dans des conditions opératoires distinctes: P₁, P₂ = Adoc = (1-adamantyloxycarbonyle, Boc (=t-butyloxycarbonyle), 2-bromo-Z (= 2-bromo-benzyloxycarbonyle), ou 2-chloro-Z (= 2-chloro-benzyloxycarbonyle) ou Fmoc ( = 9-fluorènylméthoxycarbonyle) ou Formyle ou Nicotinoyle ou 4-nitro-Z ( = 4-nitro-benzyloxycarbonyle) ou Tfa (= trifluoroacétyle) ou Tos (= p-toluènesulfonyle) ou Z (= benzyloxycarbonyle) ou Adpoc (= 1-(1-adamantyl)-1-méthylethoxycarbonyle), cette liste non exhaustive n'étant donnée qu'à titre d'exemple.

De manière tout à fait préférée, les groupes protecteurs P₁ et P₂ sont choisis de telle manière à être éliminés respectivement dans des conditions opératoires distinctes.

Le composé de formule (II) peut dans certains cas être salifié par une base, préférentiellement une base organique, et plus, préférentiellement encore une amine organique comme par exemple la dicyclohexylamine ou l'éthyldiisopropylamine. Préférentiellement, le composé de structure (II) est Boc-Lys (Z)-OH, (P₁ et P₂ ayant respectivement les valeurs Boc et Z).

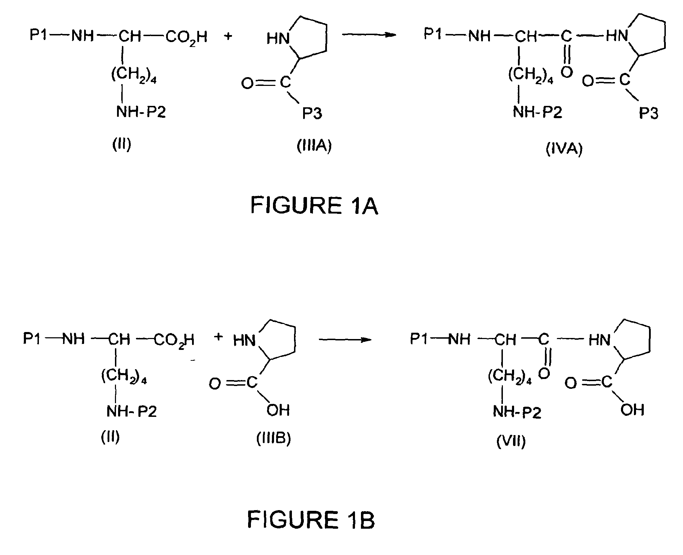

Le couplage peptidique permettant l'introduction du second acide aminé est réalisé soit avec un dérivé C-protégé de la proline de structure (IIIA) (Figure 1A) soit avec la proline de structure (IIIB) (Figure 1 B), les composés (IIIA) et (IIIB) étant tous les deux des composés de formule (III).

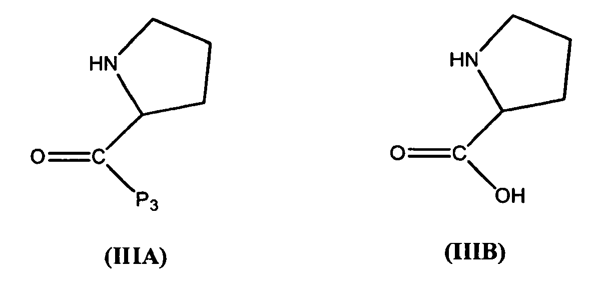

Les composés de formule (III) sont éventuellement salifiés par un acide minéral ou organique. Les composés de formule (III) sont choisis parmi H-Pro-AMC, H-Pro-p-nitrobenzylester, H-Pro-OtBu, H-Pro-OBzl, H-Pro-OMe, H-Pro-OEt préférentiellement H-Pro-OBzl ou H-Pro-OMe ou H-Pro-OEt.

Le couplage peptidique de la lysine diprotégée (II) avec la proline C-protégée (III) est réalisé à l'aide des réactifs d'activation ou de couplage classiques en synthèse peptidique comme les carbodiimides tels que la DCC (= dicyclohexylcarbodiimide) ou les formes hydrosolubles de carbodiimides telles que l'EDC (= N-éthyl-N'-(3-diméthylaminopropyl)carbodiimide chlorhydrate), des sels de phosphonium comme le BOP (= benzotriazol-1-yloxy) tris(diméthylamino)phosphonium hexafluorophosphate), le PyBOP (=(benzotriazol-1-yloxy)tripyrrolidinophosphonium hexafluorophosphate), le PyBROP (= bromotripyrrolidinophosphonium hexafluorophosphate), le PyCloP (= chlorotripyrrolidinophosphonium hexafluorophosphate), ou encore à l'aide de réactifs tels que le PyCIU (= chloro-N,N,N',N'-bis(tétraméthylène) formamidinium hexafluorophosphate), la N-hydroxysuccinimide, l'EEDQ (= 1-éthoxycarbonyl-2-éthoxy-1,2-dihydroquinoline), le CDI (= carbonyldiimidazole), ou encore des chloroformiates tels que le chloroformiate d'éthyle ou le chloroformiate d'isobutyle. Lorsque le couplage est réalisé à l'aide de réactifs de couplage tels que les carbodiimides, des additifs tels que HOBt (= 1-hydroxybenzotriazole) ou N-hydroxysuccinimide peuvent être ajoutés lors de la réaction pour limiter la racémisation.

Préférentiellement, selon le schéma de la Figure 1A, le couplage peptidique est réalisé avec des carbodiimides additionnés ou non de 1-hyd roxybenzotriazole.

Préférentiellement, selon le schéma de la Figure 1B, le couplage est réalisé avec la N-hydroxysuccinimide. Dans les deux cas, les solvants préférés sont les solvants dipolaires aprotiques, préférentiellement le diméthylformamide (DMF), la N-méthylpyrrolidone (NMP), ou des solvants aprotiques tels que le 1,2-diméthoxyéthane (DME), le tétrahydrofurane (THF), le dioxane, purs ou en mélange.

Le dernier couplage peptidique est réalisé avec la valine C-amidifiée (V) éventuellement salifiée par un acide minéral ou organique (R₂ et R₃ ayant les valeurs déjà explicitées), cet intermédiaire (V) étant lui-même préparé à partir de BOC-Val-OH par les méthodes de synthèse d'amides à partir d'acides carboxyliques.

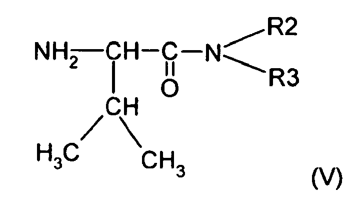

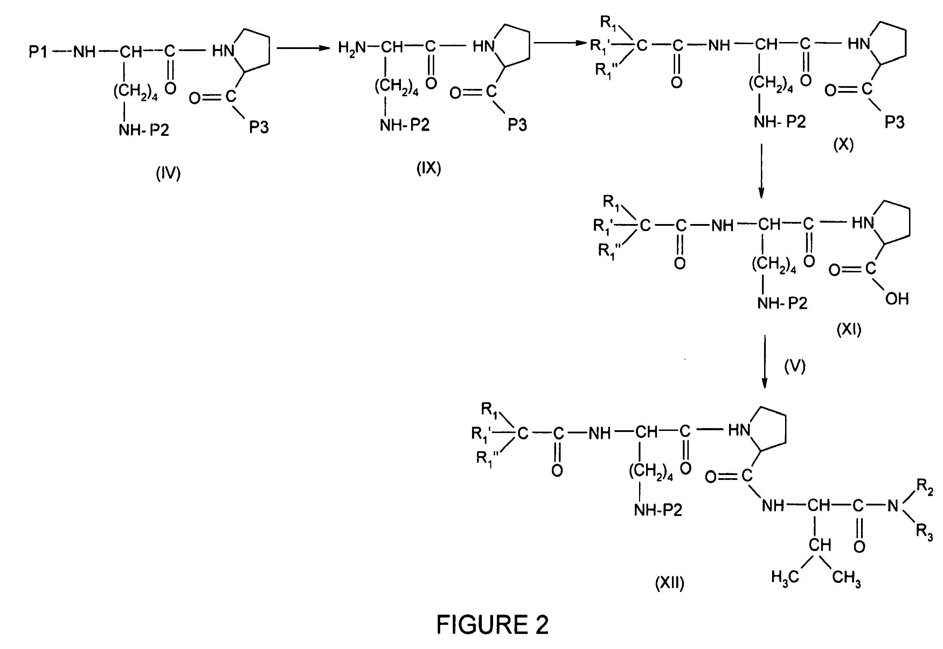

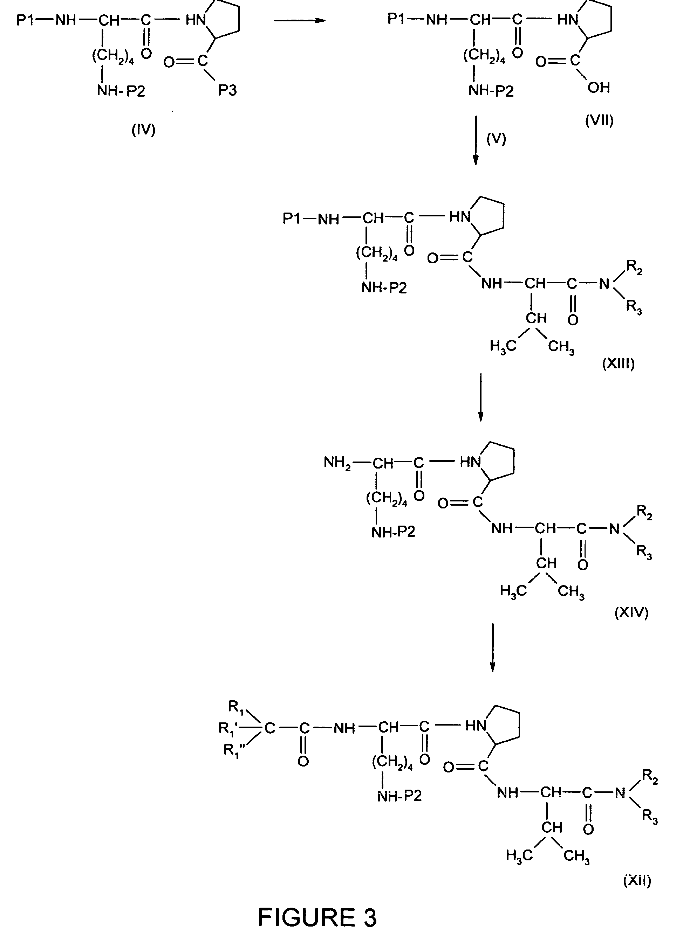

Ainsi, l'intermédiaire (IV) peut être couplé à l'intermédiaire (V) selon le schéma de la Figure 2 ou le schéma de la Figure 3.

De même, l'intermédiaire (VII) peut être couplé à (V) selon le schéma de la Figure 3, le composé (VII) ayant la formule suivante :

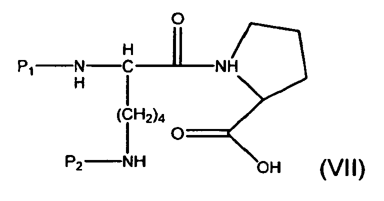

### Premier mode de réalisation préféré de l'étape b) du procédé (Figure 2).

Selon ce premier mode de réalisation préféré du procédé de synthèse des dérivés diamides du tripeptide KPV, le dipeptide KP (IV) possède un résidu de proline dont le groupe carboxylique est protégé par un groupe protecteur P3 et le couplage du dipeptide KP (IV) avec un dérivé de valine (IIIA) est réalisé après élimination du groupe protecteur P1 puis amidification de la fonction amine libre ainsi libérée NH2(α) du résidu lysine. Ce premier mode de réalisation préféré est illustré à la Figure 2.

Selon ce mode de réalisation préféré, le procédé de synthèse des dérivés diamides du tripeptide KPV, tel que défini ci-dessus, est caractérisé en ce que l'étape b) comprend les étapes suivantes :
b1) Elimination du groupe protecteur P₁ du composé de formule (IV) dans laquelle P₃ représente un groupe protecteur, de manière à obtenir le composé de formule (IX) : 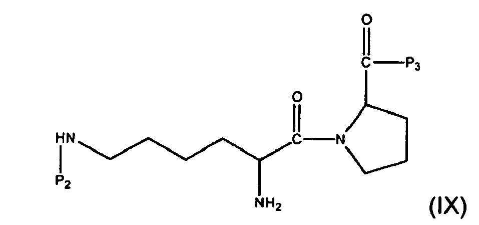 dans laquelle P₁ a la même signification que ci-dessus et P₃ représente un groupe protecteur ;
b2) amidification du groupe NH₂(α) du résidu lysine du composé de formule (IX) avec le composé de formule (VI-A) ou le composé de formule (VI-B) suivant : 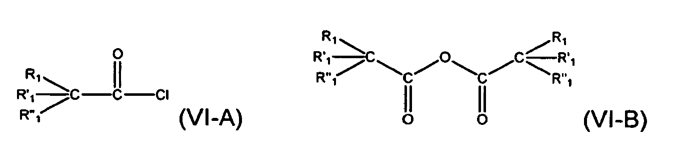 dans lesquelles R₁, R'₁ et R"₁ ont les mêmes significations que ci-dessus afin d'obtenir le composé de formule (X) suivant : 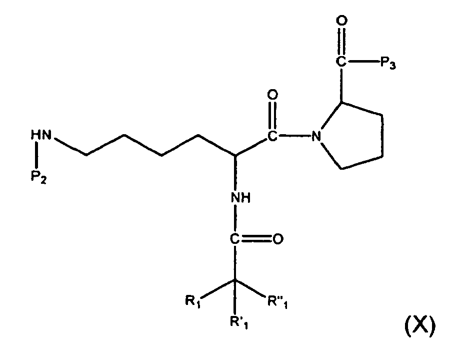 dans laquelle R₁, R'₁, R"₁, P₁ ont la même signification que ci-dessus et P3 représente un groupe protecteur ;
b3) Elimination du groupe protecteur P₃ du composé de formule (X) afin d'obtenir le composé de formule (XI) : 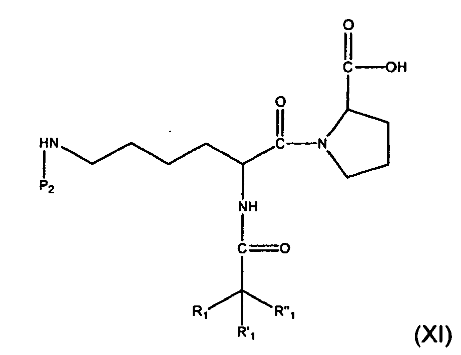 dans laquelle R₁, R'₁, R"₁ et P₂ ont la même signification que ci-dessus ;
b4) Coupler le composé de formule (XI) avec le composé de valine de formule (V) suivante, éventuellement salifié par un acide minéral ou organique : 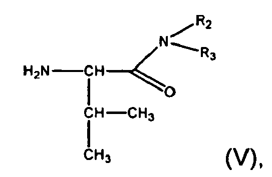 dans laquelle R₂ et R₃ ont la même signification que ci-dessus, afin d'obtenir le composé de formule (XII) suivant : 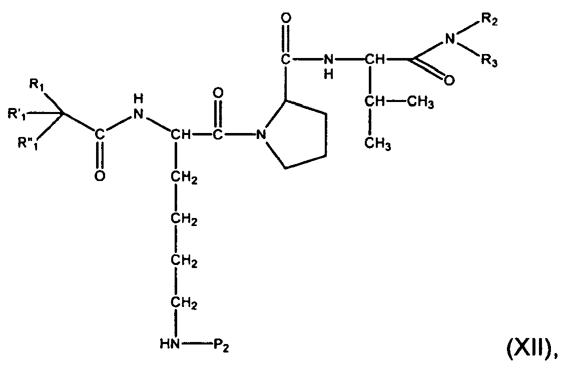 dans laquelle P₂, R₁, R'₁, R"₁, R₂ et R₃ ont la même signification que ci-dessus.

Selon le procédé illustré à la Figure 2, la fonction amine N(α) de la lysine est libérée dans les conditions nécessaires déterminées par la nature du groupe protecteur P₁ utilisé. Par exemple, si P₁ désigne le groupe BOC(=t-butyloxycarbonyle), la fonction amine N(α) de la lysine peut être libérée en traitant le composé (IV) correspondant par un acide minéral tel que l'acide chlorhydrique aqueux dans un solvant aprotique tel que le dioxane ou le THF ou encore dans un mélange de solvants tel que THF-CH₂Cl₂ ou dioxane-CH₂Cl₂. La réaction est alors réalisée entre -10°C et 40°C, préférentiellement entre 4°C et 30°C. La fonction amine N(α) de la lysine du composé (IX) est ensuite amidifiée dans les conditions usuelles comme par exemple par réaction avec un chlorure d'acide de structure (VIA), ou un anhydride symétrique de structure (VI-B), ou un anhydride dissymétrique, cette liste n'étant pas exhaustive.

Préférentiellement, la réaction est réalisée avec un chlorure d'acide (VI-A) ou un anhydride (VI-B). Le solvant est préférentiellement un solvant dipolaire aprotique tel que le DMF ou la NMP, ou un solvant aprotique tel que le THF, le dioxane, ces solvants étant utilisés purs ou en mélange, ou un mélange de solvants tel que CH₂CI₂/eau par exemple.

Si le composé (IX) est sous forme salifiée, le milieu réactionnel est additionné d'une quantité suffisante de base organique telle que la triéthylamine, l'éthyldiisopropylamine, la butylamine par exemple, ou minérale telle que NaOH, KOH, Na₂CO₃, K₂CO₃, KHCO₃, NaHCO₃ par exemple.

La fonction acide C-terminale du composé (X) ainsi obtenu est alors libérée dans les conditions opératoires nécessaires déterminées par la nature du groupe protecteur P₃. Par exemple, si P₃ désigne l'ester benzylique OBzI, la fonction acide peut être libérée en additionnant au composé (X) une base minérale ou organique, préférentiellement une base minérale telle que NaOH par exemple. Dans ce dernier cas, cette déprotection est réalisée en milieu alcoolique tel qu'éthanol ou méthanol ou en milieu hydroalcoolique à une température de -5°C au reflux, préférentiellement de +4°C à +40°C. Le composé (XI) est alors obtenu après acidification du milieu, préférentiellement par un acide minéral tel que l'acide chlorhydrique.

Le dernier couplage est alors réalisé entre le composé (XI) et le composé (V), ce dernier étant éventuellement salifié par un acide minéral ou organique.
Les méthodes de couplage sont choisies parmi celles précédemment citées. Préférentiellement, le couplage est réalisé à l'aide de sels de phosphonium tels que le BOP ou le PyBOP ou le PyBROP ou le PyCloP ou le PyCIU ou en utilisant des sels d'uronium tels que le HBPyU (= O-(benzotriazol-1-yl)-N,N,N',N'-bis(tétraméthylène)uronium hexafluorophosphate), ou le HBTU (= O-(benzotriazol-1-yl)-N,N,N',N'-tétraméthyluronium hexafluorophosphate) ou le HATU (= O-(7-azabenzotriazol-1-yl)-N-,N,N',N'-tétraméthyluronium hexafluorophosphate). Plus préférentiellement, le couplage est réalisé avec le BOP ou avec le HBTU. Ce couplage est réalisé dans un solvant dipolaire aprotique tel que le DMF ou le NMP, ou dans un solvant aprotique tel que le THF ou le dioxane. La réaction est conduite en milieu basique, la base étant préférentiellement une base organique telle que la triéthylamine, l'éthyldiisopropylamine ou le butylamine par exemple, la température étant fixée préférentiellement entre -10°C et 40°C. On obtient ainsi le composé (XII).

### Second mode de réalisation préféré de l'étape b) du procédé (figure 3).

Selon un second mode préféré de réalisation du procédé de synthèse de dérivés diamides du tripeptide KPV, le dipeptide KP possède la fonction carboxylique du résidu proline qui est protégée par un groupe protecteur P3, puis le groupe protecteur P₃ est éliminé avant couplage de la proline avec le composé de valine (V), puis le groupe protecteur P1 est éliminé et la fonction NH2(α) libre du résidu lysine est amidifiée. Le second mode de réalisation préféré du procédé est illustré à la Figure 3.

Selon ce second mode de réalisation, le procédé de synthèse de dérivés diamides du tripeptide KPV, tel que défini de manière générale dans la description est caractérisé en ce que l'étape b) comprend les étapes suivantes :
b5) Eliminer le groupe P₃ du composé de formule (IV) dans lequel le groupe P₃ représente un groupe protecteur, afin d'obtenir le composé de formule (VII) suivante : 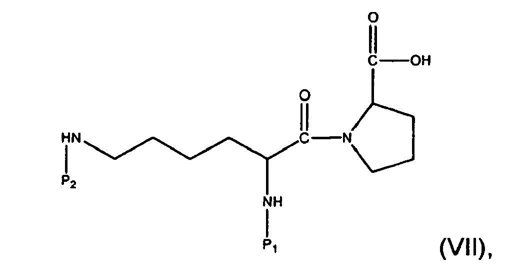 dans laquelle P₁ et P₂ ont la même signification que ci-dessus ;
b6) couplage du composé de formule (VII) avec le composé de valine de formule (V), éventuellement salifié par un acide minéral organique: 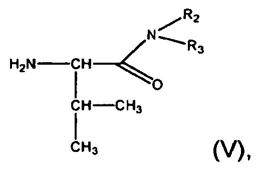 dans laquelle R₂ et R₃ ont la même signification que ci-dessus afin d'obtenir un composé de formule (XIII) : 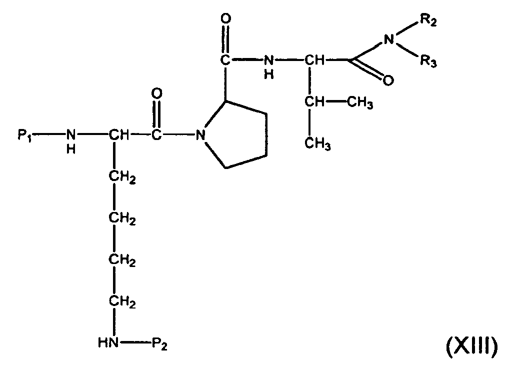 dans laquelle P₁, P₂, R₂ et R₃ ont la même signification que ci-dessus ;
b7) Eliminer le groupe protecteur P₁ du composé de formule (XIII) afin d'obtenir le composé de formule (XIV) suivant, et éventuellement salifié par un acide minéral ou organique : 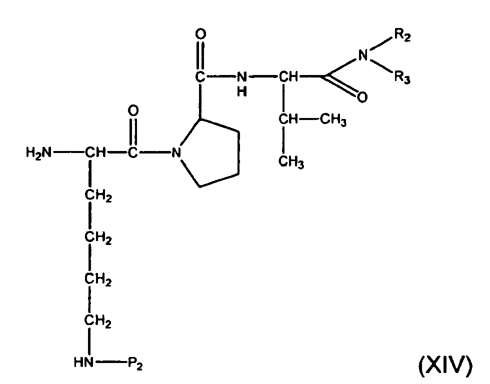 dans laquelle P₂, R₂ et R₃ ont la même signification que ci-dessus ;
b8) amidification du groupe NH₂(α) du résidu lysine du composé de formule (XIV) avec le composé de formule (VI-A) ou le composé de formule (VI-B) suivant, éventuellement salifié par un acide minéral ou organique : 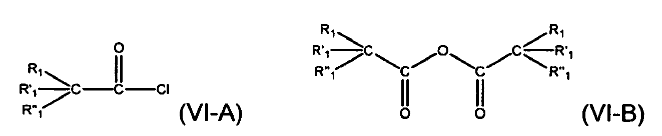 dans lesquelles R₁, R'₁ et R"₁ ont les mêmes significations que ci-dessus afin d'obtenir le composé de formule (XII) suivant : 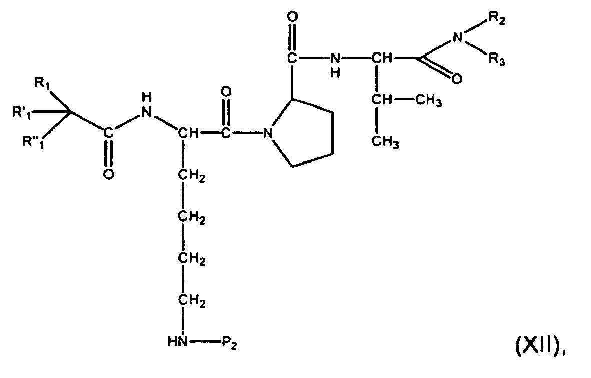 dans laquelle P₂, R₁, R'₁, R"₁, R₂ et R₃ ont la même signification que ci-dessus.

Selon le procédé illustré à la Figure 3, le dipeptide protégé (IV) est d'abord saponifié par une base minérale telle que la soude afin d'obtenir le dérivé (VII). Le couplage peptidique entre (VII) et le dérivé de valine (V) (ou un de ses sels) est réalisé par une des méthodes précédemment décrites, préférentiellement en utilisant une méthode mettant en oeuvre les carbodiimides. Plus préférentiellement, le carbodiimide utilisé est l'EDC. La réaction est alors réalisée dans un solvant dipolaire aprotique tel que le DMF ou le NMP ou un solvant aprotique comme le THF ou le dioxane, en milieu basique, par exemple par ajout d'une base organique telle que la triéthylamine ou l'éthyldiisopropylamine. La fonction amine N(α) du tripeptide (XIII) ainsi obtenu peut alors être libérée en milieu acide, par exemple par l'acide chlorhydrique. Cette déprotection est réalisée par exemple par HCI aqueux dans un mélange dioxane-dichlorométhane. Le composé (XIV) est alors obtenu sous forme de chlorhydrate. La fonction amine N(α) salifiée peut être amidifiée par exemple par réaction avec un anhydride symétrique (VI-B) en milieu basique tel qu'en présence d'éthyldiisopropylamine ou de triéthylamine, dans un solvant dipolaire aprotique tel que le DMF ou le NMP, préférentiellement le DMF, pour conduire au tripeptide (XII).

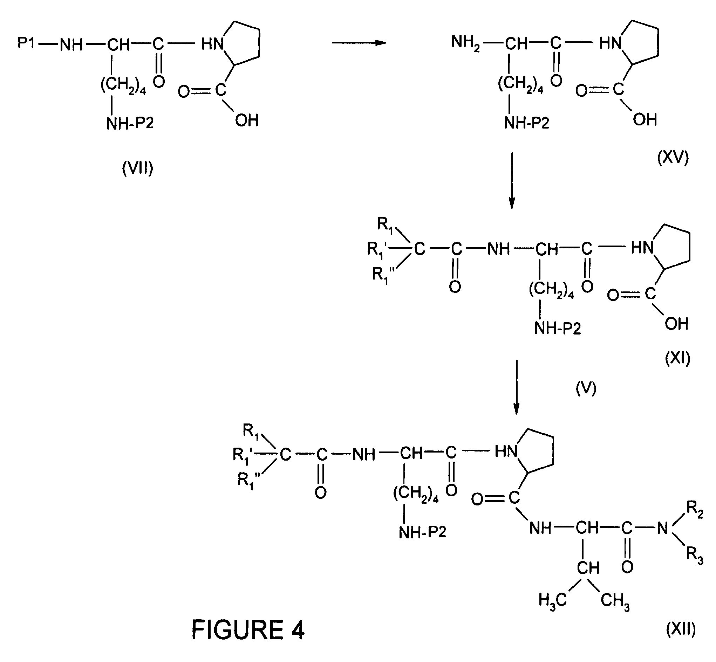

### Troisième mode de réalisation préféré de l'étape b) du procédé (figure 4).

Selon un troisième mode de réalisation du procédé, on met en oeuvre le dipeptide KP (XII) dont la fonction carboxylique du résidu proline n'est pas protégée (P₃=OH), on élimine le groupe protecteur P₁ et on amidifie le groupe NH2(α) de la lysine avant couplage du composé résultant avec le résidu valine (III). Ce troisième mode de réalisation du procédé de l'invention est illustré à la Figure 4.

Dans ce troisième mode de réalisation préféré, le procédé de synthèse de dérivés diamides du tripeptide KPV, tel que défini de manière générale dans la description, est caractérisé en ce que l'étape b) comprend les étapes suivantes :
b9) Eliminer le groupe protecteur P₁ du composé de formule (VII) afin d'obtenir le composé de formule (XV) suivant, éventuellement salifié par une base organique ou minérale : 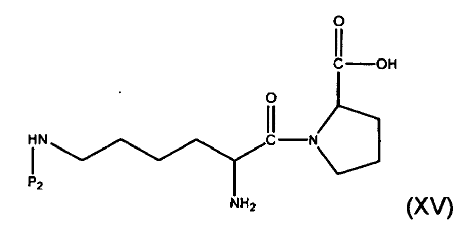 dans laquelle P₂ a la même signification que ci-dessus ;
b10) amidification du groupe NH₂(α) du résidu lysine du composé de formule (XV) avec le composé de formule (VI-A) ou le composé de formule (VI-B) suivant : 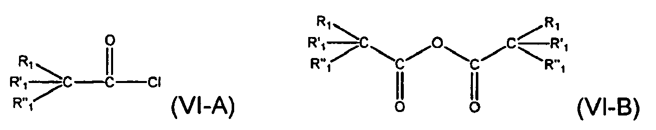 dans lesquelles R₁, R'₁ et R"₁ ont les mêmes significations que ci-dessus, afin d'obtenir le composé de formule (XI) suivant, éventuellement salifié par une base organique ou minérale : 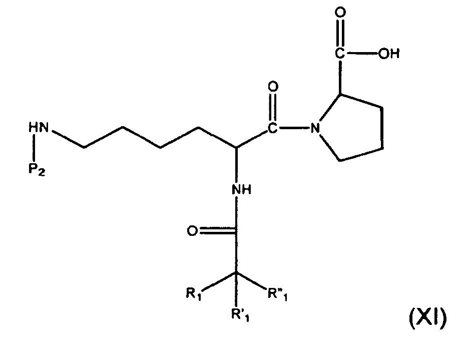 dans laquelle P₂, R₁, R'₁ et R"₁ ont la même signification que ci-dessus ;
b11) couplage du composé de formule (XI) avec le composé de valine de formule (V) suivant, éventuellement salifié par un acide minéral ou organique: 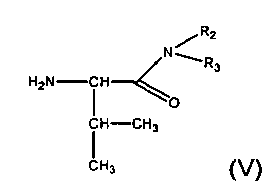 dans laquelle R₂ et R₃ ont la même signification que ci-dessus ; afin d'obtenir le composé de formule (XII) : 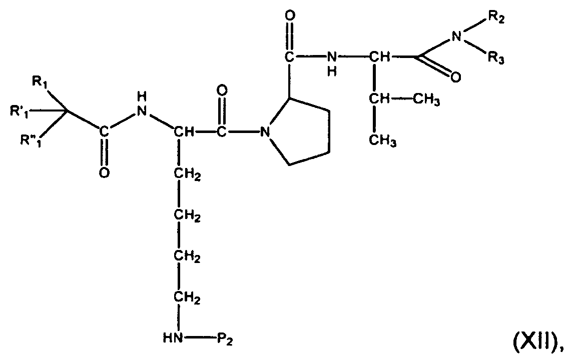 dans laquelle P₂, R₁, R'₁, R"₁, R₂ et R₃ ont la même signification que ci-dessus.

Selon le procédé illustré à la Figure 4, la fonction amine N(α) du dérivé (VII) est libérée en milieu acide, par exemple par l'acide chlorhydrique. Cette déprotection est réalisée par exemple par HCI aqueux dans un mélange dioxane-dichlorométhane pour conduire au composé (XV) salifié. La fonction amine N(α) peut alors être amidifiée comme selon la figure 3 pour conduire au dérivé (XI).

Le couplage peptidique de (XI) avec le dérivé de valine (V) (ou un de ses sels) est réalisé par une des méthodes précédemment décrites, préférentiellement en utilisant une méthode mettant en oeuvre des sels de phosphonium. Plus préférentiellement, le sel de phosphonium choisi est le BOP. La réaction est réalisée dans un solvant dipolaire aprotique tel que le DMF ou la NMP ou dans un solvant aprotique tel que le THF ou le dioxane, en milieu basique, par exemple par ajout d'une base organique telle que la triéthylamine ou l'éthyl-diisopropylamine.

### Mode de réalisation préféré de l'étape c) du procédé

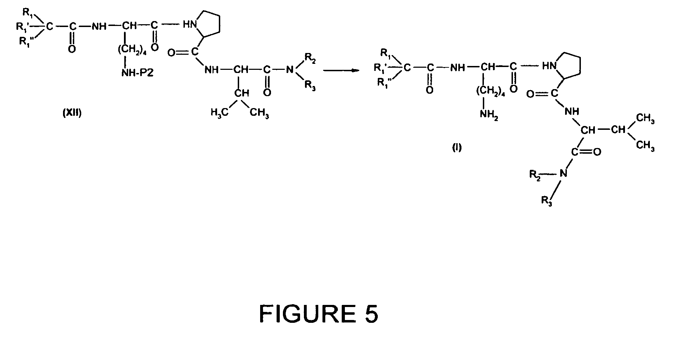

A l'étape c) du procédé, on élimine le groupe protecteur P2 du composé de formule (XII) afin d'obtenir le dérivé diamide dérivé du tripeptide KPV de formule (I), éventuellement sous la forme d'un sel. L'étape c) du procédé est illustrée à la Figure 5.

Le composé (XII) obtenu selon l'un ou l'autre des modes de réalisation illustrés dans les Figures 2, 3 ou 4, peut alors être facilement transformé en une étape pour conduire au composé (I) correspondant, cette étape étant la déprotection de la fonction amine N(ε) de la lysine, comme illustré dans la Figure 5.

Préférentiellement, la fonction amine N(ε) est protégée par un groupe benzyloxycarbonyl (Z) et la déprotection est réalisée par hydrogénation catalysée par le Palladium sur charbon. Plus préférentiellement la réaction est réalisée sous une pression d'hydrogène de 1 à 3 bars en milieu alcoolique tel que l'éthanol et en présence de 1 à 5 équivalents d'un acide organique ou minéral tel que l'acide chlorhydrique, l'acide bromhydrique, l'acide sulfurique, l'acide acétique, l'acide citrique, l'acide tartrique, l'acide lactique, l'acide phosphorique, l'acide hydrogénophosphorique, l'acide propionique ou l'acide succinique.

L'hydrogénation est préférentiellement réalisée entre 5°C et 50°C, plus préférentiellement entre 10°C et 30°C. Le composé (I) est alors directement obtenu sous forme salifiée.

### Caractéristiques techniques générales préférées du procédé

De préférence, le procédé selon l'invention est caractérisé en ce que dans le composé de formule (II), le groupe protecteur P₁ est le t-butyloxycarbonyle (Boc) et le groupe protecteur P₂ est le benzyloxycarbonyle (Z).

De préférence, le procédé selon l'invention est caractérisé en ce que dans la composé de formule (III), le groupe protecteur P₃ est le groupe ester benzylique OBzI.

De préférence, le procédé selon l'invention est caractérisé en ce que dans le composé de formule (I), les groupes R₁, R'₁ et R"₁ représentent chacun un atome d'hydrogène.

De préférence, le procédé selon l'invention est caractérisé en ce que dans le composé de formule (I), les groupes R₂ et R₃ représentent chacun un atome d'hydrogène.

De préférence, le procédé selon l'invention est caractérisé en ce que le groupe protecteur P₁ est le t-butyloxycarbonyle (Boc), le groupe protecteur P₂ est le benzyloxycarbonyle (Z) et le groupe protecteur P₃ est l'ester benzylique OBzl.

### Meilleur mode de réalisation du procédé selon l'invention

De manière tout à fait préférée, les diamides dérivés du tripeptide KPV de formule (I) sont préférentiellement obtenus à partir du composé (II) pour lequel P₁ désigne le groupe t-butyloxycarbonyle (Boc), et P₂ désigne le groupe benzyloxycarbonyle (Z) et à partir du composé (III-A) pour lequel la fonction acide est protégée par un ester benzylique, le composé (III-A) correspondant étant salifié préférentiellement par l'acide p-toluènesulfonique.

Ces composés sont préférentiellement mis en oeuvre selon l'enchaînement des voies illustrées successivement dans la Figure 1A, puis la Figure 2 (premier mode de réalisation préféré de l'étape b) du procédé), puis la Figure 5, afin d'obtenir le dérivé diamide du tripeptide KPV de formule (I).

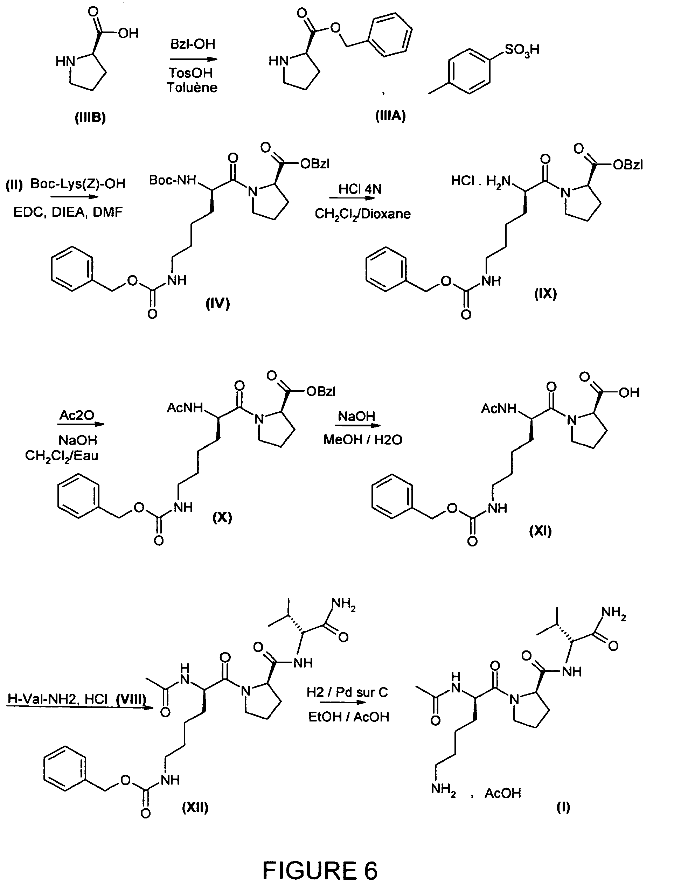

Suivant la nature des substituants R₁, R₁', R₁", R₂ et R₃, l'enchaînement des voies préféré peut permettre une mise en oeuvre ne nécessitant pas l'isolement et/ou la purification des intermédiaires. Ainsi pour R₁, R₁', R₁", R₂ et R₃ = H, le procédé plus particulièrement préféré met en oeuvre les réactifs et les conditions opératoires décrites dans la Figure 6

Selon le mode de réalisation du procédé de synthèse de dérivés diamides du tripeptide KPV illustré à la Figure 6, les composés intermédiaires (III), (IV) et (IX) ne sont pas purifiés et sont engagés tels quels dans les étapes suivantes. Ce procédé ne met en oeuvre aucune purification par colonne chromatographique ou échange d'ion. Il est applicable à tous les sels revendiqués. La pureté du produit final est mesurée par HPLC.

De manière très avantageuse, les conditions opératoires minimisent la racémisation.

### Nouveaux composés et nouvelles compositions selon l'invention

L'invention a aussi pour objet un dérivé ou sel de dérivé diamide du tripeptide KPV de formule (IA) suivante : 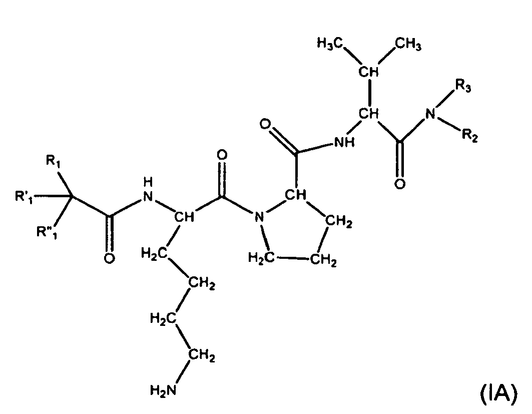 dans laquelle :
a) R₁, R'₁ et R"₁ représentent, indépendamment l'un de l'autre,
   - alkyle C₁-C₂₂, linéaire ou ramifié, éventuellement interrompu par un hétéroatome tel que O ou N ou S ou Si
   - cycloalkyle C₄-C₁₀
   - polyfluoroalkyle ou perfluoroalkyle C₁-C₂₂, linéaire ou ramifié
   - aryle éventuellement substitué par un ou plusieurs atomes d'halogène tels que CI, F , Br ou I ou, un ou plusieurs groupes alkyles C₁-C₄ linéaires ou ramifiés.
   - aralkyle
   - ou R₁ et R'₁ peuvent former avec C(R"₁) un cycle saturé de 3 à 7 atomes, éventuellement substitué par un ou plusieurs radicaux alkyles C₁-C₄ linéaires ou ramifiés et/ou renfermant éventuellement un hétéroatome tel que O, S ou N,
   - hydrogène
   à condition que le groupe R₁(R'₁)(R"₁)CO ne représente pas un résidu acide aminé ou un reste peptidique avec au moins l'un des R1, R'1, R "1 différent de l'hydrogène;
b) R₂ et R₃ représentent, indépendamment l'un de l'autre un atome d'hydrogène, ou représentent
   - alkyle C₁-C₂₄, linéaire ou ramifié, éventuellement interrompu par un hétéroatome tel que O ou N ou S ou Si
   - cycloalkyle C₄-C₁₀
   - polyfluoroalkyle ou perfluoroalkyle C₁-C₂₂, linéaire ou ramifié
   - aryle éventuellement substitué par un ou plusieurs atomes d'halogène tels que CI, F , Br ou I ou, un ou plusieurs groupes alkyles C₁-C₄ linéaires ou ramifiés.
   - aralkyle
   - ou R₂ et R₃ peuvent former avec l'atome d'azote un cycle saturé de 5 ou 6 atomes éventuellement substitué par un ou plusieurs radicaux alkyle C₁-C₄ linéaires ou ramifiés, cycle saturé renfermant éventuellement un hétéroatome tel que O, S ou encore un atome d'azote supplémentaire ,
   avec au moins l'un des résidus R2 ou R3 différents de l'hydrogène à condition que le groupe N(R₂) (R₃) ne représente pas un aminoacide ou un reste peptidique ;

De préférence, le sel ou dérivé diamide du tripeptide KPV ci-dessus est choisi parmi les chlorhydrates, bromhydrates, sulfates, acétates, citrates, tartrates, lactates, phosphates, hydrogénophosphates, propionates et succinates.

Préférentiellement, le sel de dérivé diamide du tripeptide KPV ci-dessus est caractérisé en ce que les résidus d'acides aminés Lysine, Proline ou Valine peuvent être n'importe lequel des stéréoisomères de chacun de ces résidus.

L'invention est aussi relative à des compositions contenant, dans un milieu physiologiquement acceptable, un dérivé diamide du tripeptide KPV de formule (IA) tel que défini ci-dessus.

Selon un premier mode de réalisation avantageux, les compositions ci-dessus sont caractérisées en ce que le milieu est un milieu cosmétique et que le dérivé (IA) est présent en une teneur de 10⁻⁸ à 10⁻³ g/100g.

Selon un second mode de réalisation avantageux, les compositions ci-dessus sont caractérisées en ce que le milieu est un milieu pharmaceutique et que le dérivé (IA) est présent en une teneur supérieure à 5.10⁻⁴ g/100g.

La présente invention concerne aussi l'utilisation d'un dérivé (IA) tel que défini ci-dessus dans une composition cosmétique ou pour la fabrication d'une composition dermatologique pour/ou destinée à traiter les peaux sèches et/ou les peaux sensibles.

La présente invention est en outre illustrée, sans pour autant être limitée, par l'exemple suivant.

### EXEMPLE : Synthèse du dérivé diacétylé du tripeptide KPV, sous la forme de différents sels

### I. Préparation de TosOH, H-Pro-OBzI (III):

Dans un réacteur inerte, agitable, thermostable et équipé d'un Dean-Stark, on introduit à 20°C 50.22 g d'acide p-toluène sulfonique monohydrate (1.1 eq) et 140 ml de toluène. Le milieu est agité jusqu'à dissolution et on ajoute 74.5 ml d'alcool benzylique (3 eq) puis 27.63 g de H-D-Pro-OH (240 mmoles). Le mélange est porté à reflux 16 h sous vide de 250-300 mbar. La fin de réaction est contrôlée par CCM. Le milieu est concentré sous vide jusqu'à refus.

Le produit ainsi obtenu est utilisé tel quel sans purification.

### II. Préparation de Boc-D-Lys(Z)-D-Pro-OBzI (IV):

Le résidu de l'étape précédente est repris par 83 ml de DMF, additionné de 87 g de Boc-D-Lys(Z)-OH (1 eq), puis de 35 g d'HOBt, 1 H₂O (1 eq). Le mélange est refroidi à 10°C et on ajoute, en maintenant à cette température, 54.77 g de EDC (286 mmoles, 1.25 eq) puis 59 ml d'éthyldiisopropylamine (342.96 mmoles, 1.5 eq). On laisse alors revenir à 20°C et on agite de 2 à 20 h. La fin de réaction est contrôlée par CCM.

Le milieu réactionnel est versé sur 110 ml de dichlorométhane et 220 ml d'eau, agité 15 minutes, décanté et la phase organique est relavée par 2x220 ml de solution aqueuse saturée de NaHCO₃ Les phases aqueuses sont réextraites en cascade par 83 ml de dichlorométhane. Les phases organiques sont réunies, séchées sur Na₂SO₄ et ajustées à 460 ml (par concentration ou dilution).

Cette solution de Boc-D-Lys(Z)-D-Pro-OBzl est engagée telle quelle au stade suivant.

### III. Préparation de HCI, H-D-Lys(Z)-D-Pro-OBzI (IX):

A la solution précédente refroidie à 5°C sous inertage, sont ajoutés 460 ml (17 vol.) d'HCI 4 N dans le dioxane (1828 mmoles, 8 eq). Après retour à température ambiante, l'agitation est poursuivie 2 à 3 h.

La fin de réaction est contrôlée par CCM.

Le milieu réactionnel est alors versé sous agitation sur un mélange de 270 ml d'eau et 270 g de glace. Le mélange est décanté et la phase organique est utilisée telle quelle pour le stade suivant.

### IV. Préparation de Ac-D-Lys(Z)-D-Pro-OBzl (X):

La solution chlorométhylènique de HCI, H-D-Lys(Z)-D-Pro-OBzl précédemment obtenue est refroidie à 5°C, additionnée de 135 ml d'eau, puis 32.3 ml d'anhydride acétique (1.5 eq). On ajoute ensuite Qsp pH 10 en 30 minutes à 1 h, NaOH aqueux 5 N (90 à 130 ml sont nécessaires).

Le milieu est agité à température ambiante 2h30 à 3 h, et la fin de réaction est contrôlée par CCM.

Le milieu est décanté, la phase organique est lavée à l'eau puis par une solution aqueuse saturée de NaCl (110 ml, 4 vol. à chaque fois). La phase organique est séchée sur Na2SO4 et concentrée sous vide (40°C max, 50 mbar max.).

Le résidu est utilisé tel quel dans l'étape ultérieure.

### V. Préparation de Ac-D-Lys(Z)-D-Pro-OH (XI):

Le composé Ac-D-Lys(Z)-D-Pro-OBzl obtenu à l'étape précédente (théorie : 230 mmoles) est mélangé à 313 ml de méthanol, additionné de 313 ml de NaOH 1 N (313 mmoles, 1.36 eq), et agité une nuit à température ambiante. La fin de réaction est contrôlée par CCM.

On ajoute alors de l'éther diisopropylique (313 ml, 11 vol.), le milieu est décanté et la phase aqueuse est réextraite trois fois par 3x150 ml d'éther diisopropylique. La phase aqueuse est alors versée sur un mélange 540 ml de dichlorométhane et 70 ml d'HCI 4 N aqueux (1.2 eq). Le mélange est agité 15 mn et décanté. La phase organique séparée est lavée à l'eau puis par une solution aqueuse saturée de NaCI (110 ml, 4 vol. à chaque fois). La phase organique est séchée sur Na₂SO₄ et concentrée sous vide (40°C max, 50 mbar max.). Le produit ainsi obtenu 94.25 g se présente sous forme d'une mousse (rendement 98 % calculé par rapport au Boc-D-Lys(Z)-OH engagé).

### VI. Préparation de Ac-D-Lys(Z)-D-Pro-D-Val-NH2 (XII):

Dans un réacteur inerte, thermostable et muni d'une agitation mécanique, on introduit 94.25g de Ac-D-Lys(Z)-D-Pro-OH (XI) (224.7 mmoles) puis 470 ml de DMF. On additionne alors 32.58 g de HCI . H-D-Val-NH2 (V) (213.5 mmoles, 0.95 eq) et 85.22 g d'HBTU (1 eq). Le milieu est refroidi à 5 °C, et on ajoute goutte à goutte en 15 minutes 96.6 ml d'éthyldiisopropylamine (2.5 eq). On laisse revenir à 20°C et après 1 h30 à 2 h une cristallisation abondante est observée. La fin de réaction est contrôlée par CCM. Le milieu réactionnel est alors versé sur 2350 ml d'AcOEt, agité 15 minutes, filtré et lavé successivement par 470 ml d'AcOEt et 470 ml d'éther diisopropylique. Le dérivé (XII) est séché sous vide à 25°C. On obtient : ainsi 107.2 g (92 %) de Ac-D-Lys(Z)-D-Pro-D-Val-NH2.

### VII. Préparation de AcOH, Ac-D-Lys-D-Pro-D-Val-NH2 (I):

La dernière étape consiste en l'hydrogénolyse du groupe protecteur benzyloxycarbonyle Z de la lysine. L'acide utilisé dans le milieu réactionnel se retrouvera salifiant la fonction amine libérée.

### 1) Isolé sous forme d'acétate:

107 g de Ac-D-Lys(Z)-D-Pro-D-Val-NH2 sont dissous dans 535 ml d'EtOH, additionnés de 214 ml d'AcOH et de 21.4 g de Pd/C à 10 % à 50 % d'eau. L'hydrogénation est conduite à 20°C, sous une pression d'hydrogène de 1 bar pendant 16 h. La fin de réaction est contrôlée par CCM. Le catalyseur est filtré et le filtrat est concentré sous vide jusqu'à refus. Le résidu est repris par 107 ml d'EtOH, additionné de 430 ml d'iPrOH et le mélange est chauffé à 50°C puis additionné à 50°C de 1500 ml d'éther diisopropylique ajoutés goutte à goutte. Le produit attendu cristallise. Le milieu est refroidi à 20°C et agité 1 h. Le solide est filtré et lavé par 500 ml d'éther diisopropylique avant d'être séché sous vide à 25°C pour fournir 78.3g de Ac-D-Lys-D-Pro-D-Val-NH2 sous forme de sel d'acétate (Rendement 85.4 %). La pureté du produit ainsi obtenu est contrôlée par HPLC.

Si nécessaire, le produit est "recristallisé", avec filtration 0.5 µ, dans les mêmes conditions pour fournir 71 g d'AcOH . Ac-D-Lys-D-Pro-D-Val-NH2 (Rendement purification 91 %; rendement global 74 % par rapport à Boc-D-Lys(Z)-OH).

### 2) Isolé sous forme de tartrate:

Le tartrate est obtenu en réalisant l'hydrogénolyse du groupe benzyloxycarbonyl Z en présence d'acide tartrique. L'acide L et de D-tartrique fournissent un sel comparable.
5 g de Ac-D-Lys(Z)-D-Pro-D-Val-NH2 sont mis en suspension dans 25 ml d'EtOH, additionnés de 1.48 g d'acide L-(+)-tartrique (1.02 eq.) et de 0.5 g de Pd/C à 10 % à 50 % d'eau. L'hydrogénation est conduite à 20°C, sous une pression d'hydrogène de 1 bar, pendant 16h.

La fin de réaction est contrôlée par CCM. Le produit a partiellement cristallisé dans le milieu. On ajoute alors 10 ml d'eau, le catalyseur est filtré et le filtrat concentré sous vide jusqu'à refus. Le résidu est repris par 40 ml d'iPrOH chauffé à 50°C et additionné à 50°C de 40 ml d'éthanol introduits goutte à goutte. Le produit cristallise. Le milieu est refroidi à 20°C et agité 1 h avant d'être additionné de 40 ml d'iPrOH et 50 ml d'éther diisopropylique. Le solide attendu est filtré et lavé par 50 ml d'éther diisopropylique avant d'être séché sous vide à 25°C pour fournir 4.12 g de tartrate . Ac-D-Lys-D-Pro-D-Val-NH2 (rendement 80 %). Le produit ainsi obtenu est contrôlé par HPLC. Si nécessaire, il peut être purifié comme suit.

Le produit obtenu est dissout dans 2 volumes d'éthanol et 1 volume d'eau, filtré et additionné de 4 volumes d'isopropanol. La solution est filtrée puis additionnée de 14 volumes d'éther diisopropylique filtré. Après essorage des cristaux formés et séchage à 20°C sous vide on obtient 90% de produit recristallisé et filtré.

### 3) Isolé sous forme de succinate ou de citrate:

Le protocole décrit avec l'acide tartrique peut être reproduit avec l'acide succinique ou l'acide citrique.

Si nécessaire, le produit peut être purifié avec filtration 0.5 µ en dissolvant les sels respectifs dans 2 volumes d'éthanol additionnés d'un volume d'eau, filtration puis addition de 4 volumes d'isopropanol filtrés et de 14 volumes d'éther diisopropylique filtrés.

### REFERENCES

A. EBERLE, J.L. FAUCHERE, G.I. TESSER, R. SCHWYZER, *Helvetica Chimica Acta,* 1975, Vol 58, 2106.

SULI-VARGHA H., JENEY A., LAPIS K., MEDZIHRADSZKY K., *J. Med. Chem.,* 1987, 30(3), 583-6.

SULI-VARGHA H., MEDZIHRADSZKY K., *Int. J. Pept. Protein Res.,* 1984, 23 (6), 650-6.

YASUTAKE A., POWERS J., *Biochemistry,* 1981, 20 (13), 3675-9.

R. SCHWYZER, A. COSTOPANAGIOTIS, P. SIEBER, *Helv. Chim. Acta,* 1963, 46, 870-889.

K. HOFMANN, M. WOOLNER, H. YAJIMA, G. SPUHLER, T. THOMPSON, E. SCHWARTZ, *J. Am. Chem. Soc.,* 1958, 80, 6458

K. HOFFMANN, T. THOMPSON, M. WOOLNER, G. SPUHLER, H. YAJIMA, J. CIPERA, E. SCHWARTZ, *J. Am. Chem. Soc.,* 1960, 3721.

STAPLES D., SAWYER T., MAC HADLEY E., ENGEL M., DEVAUX A., AFFHOLTER J., DARMAN P., CODY W., WILKES B., HRUBY, V., *Pepf.*; *Struct Funct., Proc. Am. Pept. Symp., 9th* (1985), 691-4.

T. SAWYER, V. HRUBY, B. WILKES, M. DRAELOS, E. Mac HADLEY, M. BERGSNEIDER, *J. Med. Chem.,* 1982,25,1022-1027.

T. SAWYER, Thèse, University of Arizona, 1981

## Revendications

1. Procédé de synthèse en solution d'un dérivé diamide du tripeptide KPV de formule (I) suivante, ou d'un sel de composé de formule (I) : 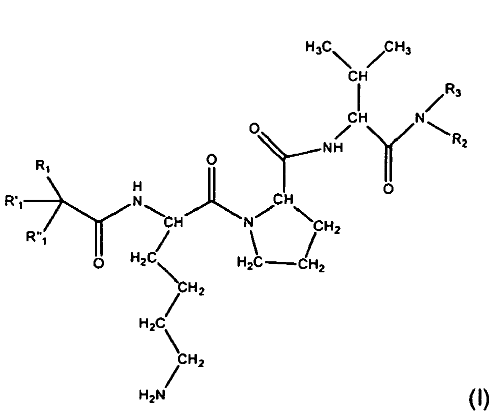 indépendamment de la stéréochimie des acides aminés mis en oeuvre, dans laquelle :
a) R₁, R'₁ et R"₁ représentent, indépendamment l'un de l'autre, un atome d'hydrogène ou
- alkyle C₁-C₂₂, linéaire ou ramifié, éventuellement interrompu par un hétéroatome tel que O ou N ou S ou Si
- cycloalkyle C₄-C₁₀
- polyfluoroalkyle ou perfluoroalkyle C₁-C₂₂, linéaire ou ramifié
- aryle éventuellement substitué par un ou plusieurs atomes d'halogène tels que Cl, F , Br ou I ou, un ou plusieurs groupes alkyles C₁-C₄ linéaires ou ramifiés.
- aralkyle
- ou R₁ et R'₁ peuvent former avec C(R"₁) un cycle saturé de 3 à 7 atomes, éventuellement substitué par un ou plusieurs radicaux alkyles C₁-C₄ linéaires ou ramifiés et/ou renfermant éventuellement un hétéroatome tel que O, S ou N,
à condition que le groupe R₁(R'₁)(R"₁)CO ne représente pas un résidu acide aminé ou un reste peptidique ;
b) R₂ et R₃ représentent, indépendamment l'un de l'autre un atome d'hydrogène, ou représentent
- alkyle C₁-C₂₄, linéaire ou ramifié, éventuellement interrompu par un hétéroatome tel que O ou N ou S ou Si
- cycloalkyle C₄-C₁₀
- polyfluoroalkyle ou perfluoroalkyle C₁-C₂₂, linéaire ou ramifié
- aryle éventuellement substitué par un ou plusieurs atomes d'halogène tels que CI, F , Br ou I ou, un ou plusieurs groupes alkyles C₁-C₄ linéaires ou ramifiés.
- aralkyle
- ou R₂ et R₃ peuvent former avec l'atome d'azote un cycle saturé de 5 ou 6 atomes éventuellement substitué par un ou plusieurs radicaux alkyle C₁-C₄ linéaires ou ramifiés, cycle saturé renfermant éventuellement un hétéroatome tel que O, S ou encore un atome d'azote supplémentaire,
à condition que le groupe N(R₂) (R₃) ne représente pas un aminoacide ou un reste peptidique ;
ledit procédé étant **caractérisé en ce qu'**il comprend les étapes suivantes :
a) réaction d'un résidu de lysine diprotégé de formule (II) suivante : 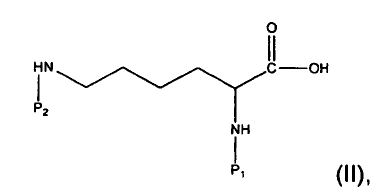 éventuellement salifié par une base minérale ou organique,
dans laquelle P₁ et P₂, différents l'un de l'autre, représentent chacun indépendamment l'un de l'autre un groupe protecteur,
avec un résidu de Proline de formule (III) suivante : 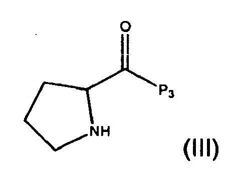 éventuellement salifié par un acide minéral ou organique,
dans laquelle P₃ représente un groupe protecteur différent de l'un quelconque des groupes protecteurs P₁ et P₂, ou dans laquelle P₃ représente un groupe hydroxyle,
en présence d'un réactif d'activation ou d'un réactif de couplage dans un solvant, afin d'obtenir le composé de formule (IV) suivant : 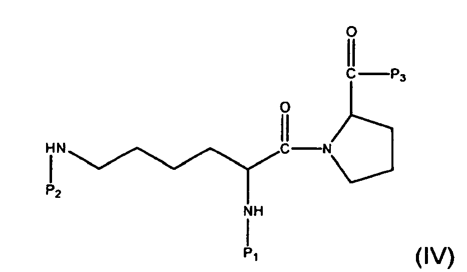 dans laquelle P₁, P₂ et P₃ ont les significations ci-dessus.
b)
1) couplage, sur la fonction C-terminale du résidu Proline du composé de formule (IV) dans lequel P3 représente OH, d'un composé de valine de formule (V) suivante, éventuellement salifié par un acide minéral ou organique: 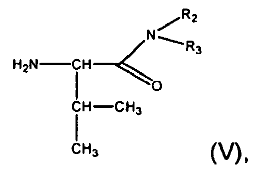 dans laquelle R₂ et R₃ ont la même signification que ci-dessus, élimination du groupe protecteur P₁;
2) amidification du groupe NH₂(α) en position N-terminale du résidu lysine par un composé de formule (VI-A) ou (VI-B) suivante 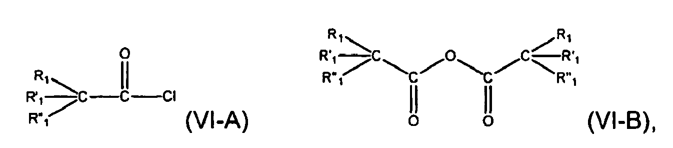 afin d'obtenir le composé de formule (XII) suivant : 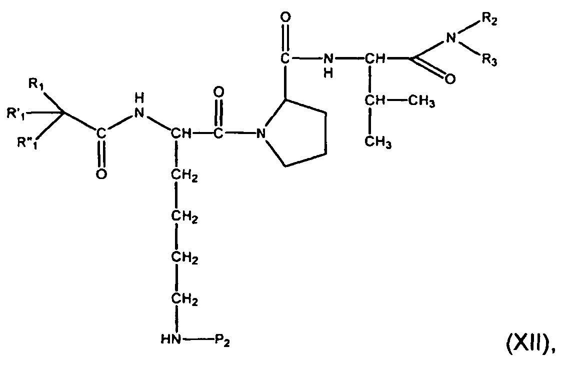 dans laquelle P₂, R₁, R'₁, R"₁, R₂ et R₃ ont la même signification que ci-dessus ;
l'ordre des étapes 1) et 2) étant indifférent;
c) Eliminer le groupe protecteur P₂ du composé de formule (XII) afin d'obtenir le composé de formule (I), éventuellement sous la forme d'un sel minéral ou organique.

2. Procédé selon la revendication 1, **caractérisé en ce que** le sel est choisi parmi les chlorhydrates, bromhydrates, sulfates, acétates, citrates, tartrates, lactates, phosphates, hydrogénophosphates, propionates et succinates.

3. Procédé selon la revendication 1 ou 2, **caractérisé en ce que** les résidus d'acides aminés Lysine, Proline ou Valine peuvent être n'importe lequel des stéréoisomères de chacun de ces résidus.

4. Procédé selon l'une des revendications 1 à 3 , **caractérisé en ce que** le sel est obtenu au cours de l'étape c) par introduction de l'acide correspondant.

5. Procédé selon la revendication 4 **caractérisé en ce que** l'acide est l'acide acétique ou chlorhydrique ou bromhydrique ou sulfurique ou citrique ou tartrique ou lactique ou phosphorique ou hydrogénophosphorique ou propionique ou succinique.

6. Procédé selon la revendication 5 **caractérisé en ce que** l'acide est l'acide acétique ou chlorhydrique.

7. Procédé selon l'une quelconque des revendications précédentes, **caractérisé en ce que** les groupes protecteurs P₁ et P₂ représentent, indépendamment l'un de l'autre Adoc (=1-adamantyloxycarbonyle) BOC (=t-butyloxycarbonyle), 2-bromo-Z (=2-bromo-benzyloxycarbonyle), ou 2-chloro-Z (=2-chloro-benzyloxycarbonyle) ou Fmoc (= 9-fluorenylméthoxycarbonyle) ou Formyle ou Nicotinoyle ou 4-nitro-Z, (= 4-nitro-benzyloxycarbonyle) ou Tfa (= trifluoroacétyle) ou Tos (= p-toluènesulfonyle) ou Z (=benzyloxycarbonyle) ou Adpoc (=1-(adamantyl)-1-méthyléthoxycarbonyle.

8. Procédé selon la revendication 7, **caractérisé en ce que** les groupes protecteurs P₁ et P₂ sont choisis de telle manière à être éliminés respectivement dans des conditions opératoires distinctes.

9. Procédé selon l'une quelconque des revendications précédentes, **caractérisé en ce que** le composé de formule (II) est salifié par une base organique, préférentiellement une amine organique.

10. Procédé selon l'une quelconque des revendications précédentes, **caractérisé en ce que** le composé de formule (III) est salifié par un acide minéral ou organique.

11. Procédé selon l'une quelconque des revendications précédentes, **caractérisé en ce qu'**à l'étape a), la réaction de couplage est réalisée en présence d'un réactif de couplage ou d'activation choisi parmi les carbodiimides tels que la DCC (= dicyclohexylcarbodiimide) ou les formes hydrosolubles de carbodiimides telles que l'EDC (= N-éthyl-N'-(3-diméthylaminopropyl)carbodiimide chlorhydrate), des sels de phosphonium comme le BOP (= benzotriazol-1-yloxy) tris(diméthylamino)phosphonium hexafluorophosphate), le PyBOP (=(benzotriazol-1-yloxy)tripyrrolidinophosphonium hexafluorophosphate), le PyBROP (= bromotripyrrolidinophosphonium hexafluorophosphate), le PyCloP (= chlorotripyrrolidinophosphonium hexafluorophosphate), ou encore à l'aide de réactifs tels que le PyClU (= chloro-N,N,N',N'-bis(tétraméthylène) formamidinium hexafluorophosphate), la N-hydroxysuccinimide, l'EEDQ (= 1-éthoxycarbonyl-2-éthoxy-1,2-dihydroquinoline), le CDI (=carbonyldiimidazole), ou encore des chloroformiates tels que le chloroformiate d'éthyle ou le chloroformiate d'isobutyle.

12. Procédé selon l'une quelconque des revendications précédentes, **caractérisé en ce que** l'étape b) comprend les étapes suivantes :
b1) Elimination du groupe protecteur P₁ du composé de formule (IV) dans laquelle P₃ représente un groupe protecteur, de manière à obtenir le composé de formule (IX) : 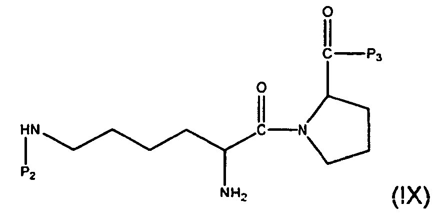 dans laquelle P₁ a la même signification que dans la revendication 1 et P₃ représente un groupe protecteur ;
b2) amidification du groupe NH₂(α) du résidu lysine du composé de formule (IX) avec le composé de formule (VI-A) ou le composé de formule (VI-B) suivant: 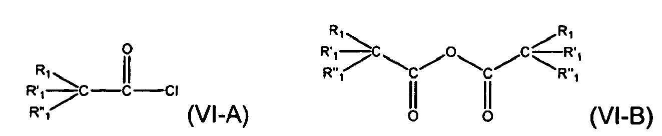 dans lesquelles R₁, R'₁ et R"₁ ont les mêmes significations que dans la revendication 1,
afin d'obtenir le composé de formule (X) suivant : 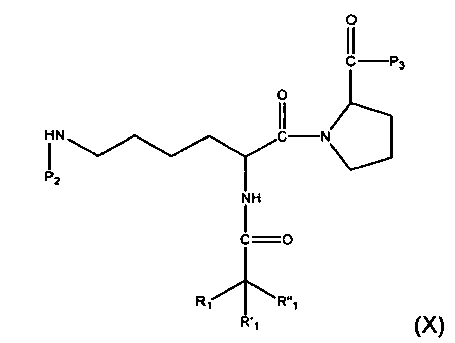 dans laquelle R₁, R'₁, R"₁, P₁ ont la même signification que dans la revendication 1 et P₃ représente un groupe protecteur ;
b3) Elimination du groupe protecteur P₃ du composé de formule (X) afin d'obtenir le composé de formule (XI) : 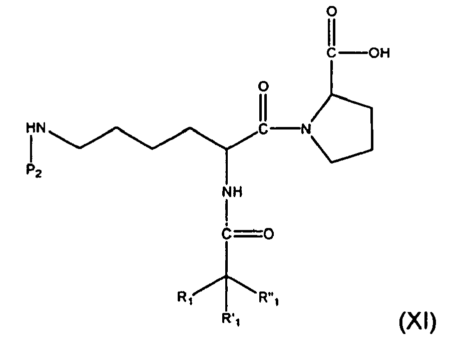 dans laquelle : R₁, R'₁, R"₁ et P₂ ont la même signification que dans la revendication 1 ;
b4) Coupler le composé de formule (XI) avec le composé de valine de formule (V) suivante , éventuellement salifié par un acide minéral ou organique: 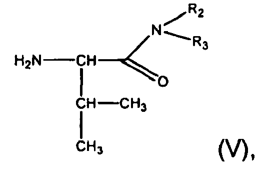 dans laquelle R2 et R3 ont la même signification que ci-dessus, afin d'obtenir le composé de formule (XII) suivant : 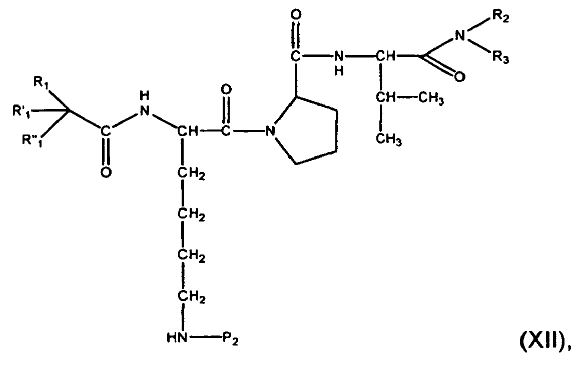 dans laquelle P₂, R₁, R'₁, R"₁, R₂ et R₃ ont la même signification que ci-dessus.

13. Procédé selon l'une des revendications 1 à 11, **caractérisé en ce que** l'étape b) comprend les étapes suivantes :
b5) Eliminer le groupe P₃ du composé de formule (IV) dans lequel le groupe P₃ représente un groupe protecteur, afin d'obtenir le composé de formule (VII) suivante : 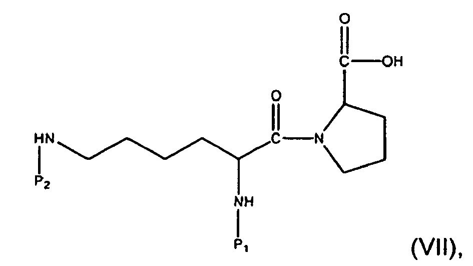 dans laquelle P₁ et P₂ ont la même signification que dans la revendication 2;
b6) couplage du composé de formule (VII) avec le composé de valine de formule (V), éventuellement salifié par un acide minéral ou organique : 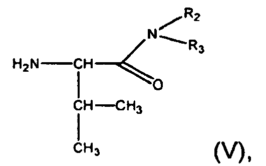 dans laquelle R₂ et R₃ ont la même signification que dans la revendication 1 afin d'obtenir un composé de formule (XIII) : 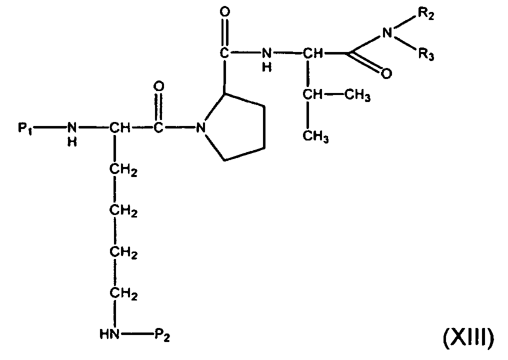 dans laquelle P₁, P₂, R₂ et R₃ ont la même signification que ci-dessus ;
b7) Eliminer le groupe protecteur P₁ du composé de formule (XIII) afin d'obtenir le composé de formule (XIV) suivant, éventuellement salifié par un acide minéral ou organique : 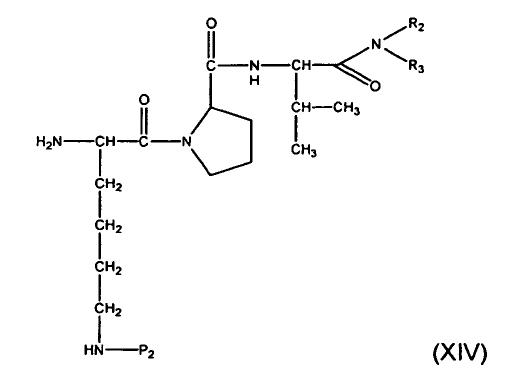 dans laquelle P₂, R₂ et R₃ ont la même signification que ci-dessus ;
b8) amidification du groupe NH₂(α) du résidu lysine du composé de formule (XIV) avec le composé de formule (VI-A) ou le composé de formule (VI-B) suivant, éventuellement salifié par un acide minéral ou organique : 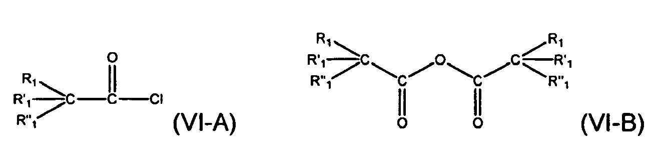 dans lesquelles R₁, R'₁ et R"₁ ont les mêmes significations que dans la revendication 1,
afin d'obtenir le composé de formule (XII) suivant : 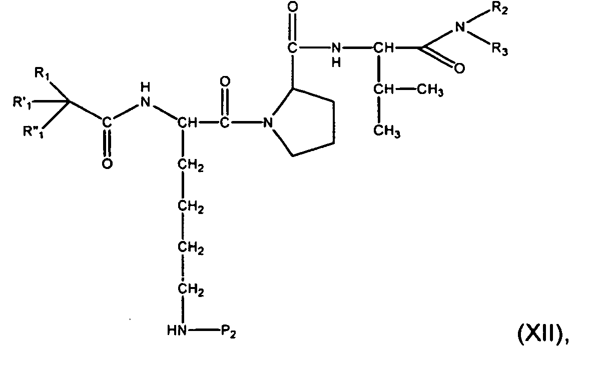 dans laquelle P₂, R₁, R'₁, R"₁, R₂ et R₃ ont la même signification que ci-dessus.

14. Procédé selon l'une des revendications 1 à 11, **caractérisé en ce que** l'étape b) comprend les étapes suivantes :
b9) Eliminer le groupe protecteur P₁ du composé de formule (VII) dans lequel le groupe P₃ représente un groupe hydroxyle afin d'obtenir le composé de formule (XV) suivant : 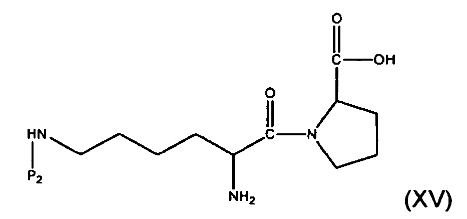 dans laquelle P₂ a la même signification que dans la revendication 2 ;
b10) amidification du groupe NH₂(α) du résidu lysine du composé de formule (XV) avec le composé de formule (VI-A) ou le composé de formule (VI-B) suivant : 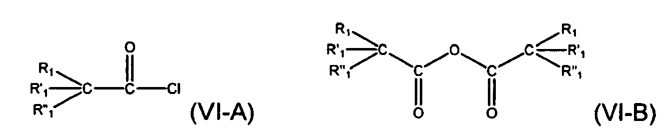 dans lesquelles R₁, R'₁ et R"₁ ont les mêmes significations que dans la revendication 1,
afin d'obtenir le composé de formule (XI) suivant, éventuellement salifié par une base organique ou minérale: 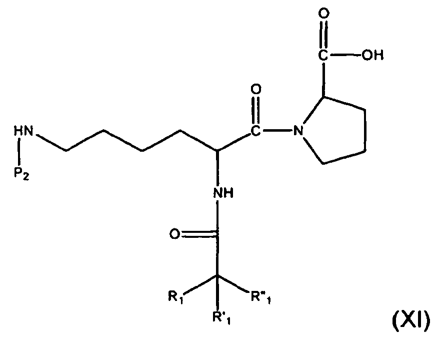 dans laquelle P₂, R₁, R'₁ et R"₁ ont la même signification que ci-dessus ;
b11) couplage du composé de formule (XI) avec le composé de valine de formule (V) suivant, éventuellement salifié par un acide minéral ou organique: 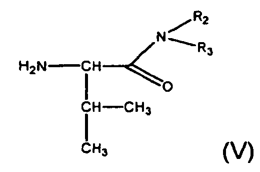 dans laquelle R₂ et R₃ ont la même signification que dans la revendication 1 ;
afin d'obtenir le composé de formule (XII) : 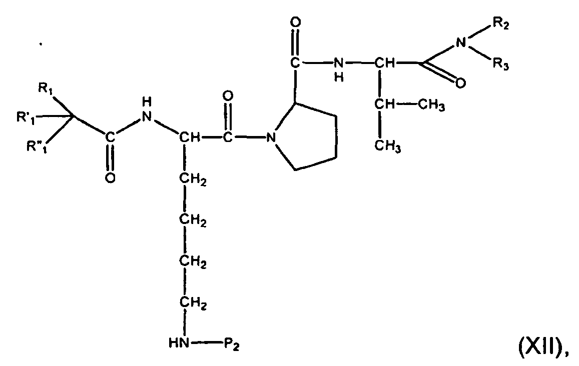 dans laquelle P₂, R₁, R'₁, R"₁, R₂ et R₃ ont la même signification que ci-dessus.

15. Procédé selon l'une des revendications 1 à 14, **caractérisé en ce que** dans le composé de formule (II), le groupe protecteur P₁ est le t-butyloxycarbonyle (BOC) et le groupe protecteur P₂ est le benzyloxycarbonyle (Z).

16. Procédé selon l'une des revendications 1 à 15, **caractérisé en ce que** dans le composé de formule (III), le groupe protecteur P₃ est le groupe ester benzylique OBzl.

17. Procédé selon l'une des revendications 1 à 16, **caractérisé en ce que** dans le composé de formule (I), les groupes R₁, R'₁ et R"₁ représentent chacun un atome d'hydrogène.

18. Procédé selon l'une des revendications 1 à 14, **caractérisé en ce que** dans le composé de formule (I), les groupes R₂ et R₃ représentent chacun un atome d'hydrogène.

19. Procédé selon l'une des revendications 1 à 14, **caractérisée en ce que** le groupe protecteur P₁ est le t-butyloxycarbonyle (BOC), le groupe protecteur P₂ est le benzyloxycarbonyle (Z) et le groupe protecteur P₃ est l'ester benzylique OBzl.

20. Dérivé ou sel de dérivé diamide du tripéptide KPV de formule (IA) suivante : 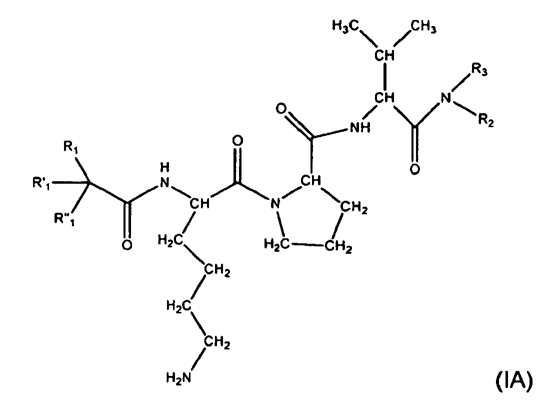 dans laquelle :
a) R₁, R'₁ et R"₁ représentent, indépendamment l'un de l'autre,
- alkyle C₁-C₂₂, linéaire ou ramifié, éventuellement interrompu par un hétéroatome tel que O ou N ou S ou Si
- cycloalkyle C₄-C₁₀
- polyfluoroalkyle ou perfluoroalkyle C₁-C₂₂, linéaire ou ramifié
- aryle éventuellement substitué par un ou plusieurs atomes d'halogène tels que Cl, F , Br ou I ou, un ou plusieurs groupes alkyles C₁-C₄ linéaires ou ramifiés.
- aralkyle
- ou R₁ et R'₁ peuvent former avec C(R"₁) un cycle saturé de 3 à 7 atomes, éventuellement substitué par un ou plusieurs radicaux alkyles C₁-C₄ linéaires ou ramifiés et/ou renfermant éventuellement un hétéroatome tel que O, S ou N,
- hydrogène
à condition que le groupe R₁(R'₁)(R"₁)CO ne représente pas un résidu acide aminé ou un reste peptidique avec au moins l'un des R1, R'1, R"1 différent de l'hydrogène;
b) R₂ et R₃ représentent, indépendamment l'un de l'autre un atome d'hydrogène, ou représentent
- alkyle C₁-C₂₄, linéaire ou ramifié, éventuellement interrompu par un hétéroatome tel que O ou N ou S ou Si
- cycloalkyle C₄-C₁₀
- polyfluoroalkyle ou perfluoroalkyle C₁-C₂₂, linéaire ou ramifié
- aryle éventuellement substitué par un ou plusieurs atomes d'halogène tels que Cl, F , Br ou I ou, un ou plusieurs groupes alkyles C₁-C₄ linéaires ou ramifiés.
- aralkyle
- ou R₂ et R₃ peuvent former avec l'atome d'azote un cycle saturé de 5 ou 6 atomes éventuellement substitué par un ou plusieurs radicaux alkyle C₁-C₄ linéaires ou ramifiés, cycle saturé renfermant éventuellement un hétéroatome tel que O, S ou encore un atome d'azote supplémentaire ,
avec au moins l'un des résidus R2 ou R3 différents de l'hydrogène
à condition que le groupe N(R₂) (R₃) ne représente pas un aminoacide ou un reste peptidique ;

21. Sel de dérivé diamide du tripeptide KPV selon la revendication 20, **caractérisé en ce que** le sel est choisi parmi les chlorhydrates, bromhydrates, sulfates, acétates, citrates, tartrates, lactates, phosphates, hydrogénophosphates, propionates et succinates.

22. Dérivé ou sel de dérivé diamide du tripeptide KPV selon la revendication 20 ou 21, **caractérisé en ce que** les résidus d'acides aminés Lysine, Proline ou Valine peuvent être n'importe lequel des stéréoisomères de chacun de ces résidus.

23. Compositions contenant dans un milieu physiologiquement acceptable un dérivé (IA) conforme à l'une quelconque des revendications 20 à 22.

24. Compositions selon la revendication 23 **caractérisée en ce que** le milieu est un milieu cosmétique et que le dérivé (IA) est présent en une teneur de 10⁻⁸ à 10⁻³ g/100g.

25. Composition selon la revendication 23 **caractérisée en ce que** le milieu est un milieu pharmaceutique et que le dérivé (IA) est présent en une teneur supérieure à 5.10⁻⁴g/100g.

26. Utilisation d'un dérivé (IA) conforme à l'une quelconque des revendications 20 à 22 dans une composition cosmétique ou pour la fabrication d'une composition dermatologique pour/ou destinée à traiter les peaux sèches et/ou les peaux sensibles.

## Claims

1. A process for synthesizing in solution a diamido derivative of KPV tripeptide having the following formula (I), or a salt of the compound of formula (I) : 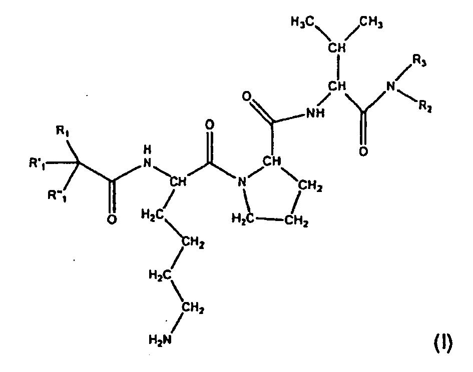 independently of the stereochemistry of the used amino acids, in which :
a) R₁, R'₁ and R"₁ are, independently from each other, a hydrogen atom, or
- a linear or branched C₁-C₂₂ alkyl, possibly interrupted by a heteroatom such as O or N or S or Si,
- a C₄-C₁₀ cycloalkyl,
- a linear or branched C₁-C₂₂ polyfluoroalkyl, or perfluoroalkyl
- an aryl possibly substituted by one or more halogen atoms such as Cl, F, Br or I or, one or more linear or branched C₁-C₄ alkyl groups
- an aralkyl;
or R₁ and R'₁ may form with C(R"₁) a saturated cycle with 3 to 7 atoms, possibly substituted by one or more linear or branched C₁-C₄ alkyl radicals and/or possibly including a heteroatom such as O, S or N, with the proviso that a group R₁(R'₁)(R"₁)CO is not an amino acid moiety or a peptidic moiety;
- R₂ and R₃ are, independently from each other, a hydrogen atom, or
- a linear or branched C₁-C₂₄ alkyl, possibly interrupted by a heteroatom such as O or N or S or Si,
- a C₄-C₁₀ cycloalkyl,
- a linear or branched C₁-C₂₂ polyfluoroalkyl or perfluoroalkyl,
- an aryl, possibly substituted by one or more halogen such as Cl, F, Br or I or, one or more linear or branched C₁-C₄ alkyl groups,
- an aralkyl ,
- or R₂ and R₃ may form with the nitrogen atom a saturated cycle having 5 to 6 atoms, possibly substituted by one or more linear or branched C₁₋C₄ alkyl radicals, said saturated cycle possibly including a heteroatom such as O, S or still an additional nitrogen atom, with the proviso that the N(R₂)(R₃) group is not an aminoacid or a peptidic moiety ;
- said process being **characterised in that** it comprises the following steps:
- reacting a diprotected lysine moiety having the following formula (II): 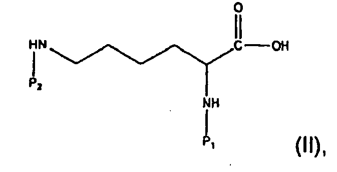
possibly in the form of a mineral or organic base salt,
in which P₁ and P₂, different from each other, are each independently from each other a protective group,
with a Proline moiety having the following formula (III) : 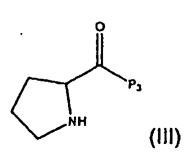 possibly in the form of a mineral or organic acid salt, in which P₃ is a protective group different from anyone of the protective groups P₁ and P₂, or in which P₃ is a hydroxyl group,
in the presence of an activating reagent or a coupling reagent in a solvent, to obtain the compound having the following formula (IV) : 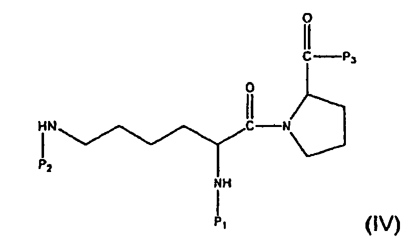 in which P₁, P₂ and P₃ have the above meanings .
b)
1) coupling, on the C-terminal function or the Proline moiety of the compound of formula (IV) in which P₃ is OH, of a valine compound having the following formula (V), possibly in the form of a mineral or organic acid salt: 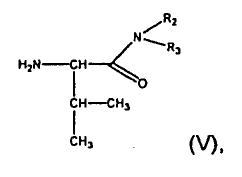
in which R₂ and R₃ have the same meanings as above, withdrawing the protective group P₁;
2) amidification of the NH₂(α) group in N-terminal position of the lysine moiety with a compound having the following formula (VI-A) or (VI-B) 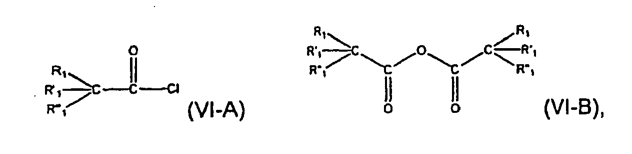 to obtain the following compound of formula (XII) : 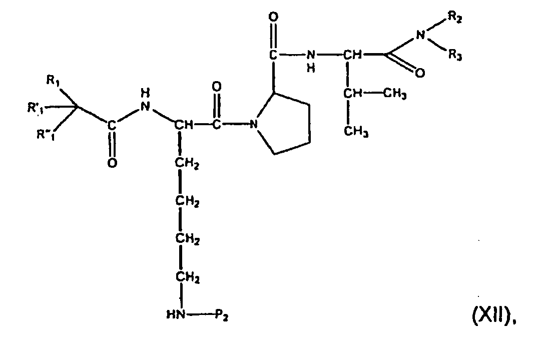 in which P₂, R₁, R'₁, R"₁, R₂ and R₃ have the same meanings as above; the order of steps 1) and 2) being indifferent;
c) withdrawing the protective group P₂ of the compound of formula (XII) to obtain the compound of formula (I), possibly in the form of a mineral or organic salt.

2. The process according to claim 1, **characterized in that** the salt is selected from chlorhydrates, bromhydrates, sulphates, acetates, citrates, tartrates, lactates, phosphates, hydrogenophosphates, propionates and succinates.

3. The process according to claim 1 or 2, **characterized in that** the Lysine, Proline or Valine aminoacids moieties can be anyone of the stereoisomers of each of these moieties.

4. The process according to anyone of claims 1 to 3, **characterized in that** the salt is obtained during the course of step c) by introduction of the corresponding acid.

5. The process according to claim 4, **characterized in that** the acid is acetic acid or hydrochloride acid or hydrobromide acid or sulphuric acid or citric acid or tartaric acid or lactic acid or phosphoric acid or hydrogenophosphoric acid or propionic acid or succinic acid.

6. The process according to claim 5, **characterized in that** the acid is acetic acid or hydrochloride acid.

7. The process according to anyone of the preceding claims, **characterized in that** the protective groups P₁ and P₂ are, independently from each other Adoc (= 1-adamantyloxycarbonyl) BOC (= t-butyloxycarbonyl), 2-bromo-Z (= 2-bromo-benzyloxycarbonyl), or 2-chloro-Z (= 2-chloro-benzyloxycarbonyl) or Fmoc ( = 9-fluorenylmethoxycarbonyl)or Formyl or Nicotinoyl or 4-nitro-Z, (= 4-nitrobenzyloxycarbonyl) or Tfa (= trifluoroacetyl) or Tos (= p-toluenesulfonyl) or Z (= benzyloxycarbonyl) or Adpoc (= 1-(adamantyl)-1-methylethoxycarbonyl.

8. The process according to claim 7, **characterized in that** the protective groups P₁ and P₂ are selected in such a way that they are respectively eliminated under distinct operating conditions.

9. The process according to anyone of the preceding claims, **characterized in that** the compounds of formula (II) is in the form of an organic base salt, preferably an organic amine.

10. The process according to anyone of the preceding claims, **characterized in that** the compound of formula (III) is in the form of a mineral or organic acid salt.

11. The process according to anyone of the preceding claims, **characterized in that** in step a), the coupling reaction is implemented in the presence of a coupling or activating reagent selected from carbodiimides such as DCC ( = dicyclohexylcarbodiimide) or hydro soluble forms of carbodiimides such as EDC ( = N-ethyl-N'-(3-dimethylaminopropyl) carbodiimide chlorhydrate), phosphonium salts such as BOP ( = benzotriazol-1-yloxy) tris(dimethylamino)phosphonium hexafluorophosphate), PyBOP (= (benzotriazol-1-yloxy)tripyrrolidinophosphonium hexafluorophosphate), PyBROP (=bromotripyrrolidinophosphonium hexafluorophosphate), PyCloP (=chlorotripyrrolidinophosphonium hexafluorophosphate), or still with reagent such as PyCIU (=chloro-N,N,N',N'-bis(tetramethylen) formamidinium hexafluorophosphate), N-hydroxysuccinimide, EEDQ (=1-ethoxycarbonyl-2-ethoxy-1,2-dihydroquinoline, CDI (=carbonyldiimidazole), or still chloroformates such as ethyl chloroformate or isobutyl chloroformate.

12. The process according to anyone of the preceding claims, **characterized in that** step b) comprises the following steps:
b1) Withdrawal of protective group P₁ from compound of formula (IV) in which P₃ is a protective group, to obtain a compound of formula (IX): 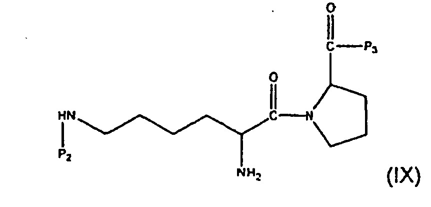 in which P₁ has the same meaning as in claim 1 and P₃ is a protective group;
b2) amidification of group NH₂(α) of the lysine moiety of the compound of formula (IX) with the following compound of formula (VI-A) or the following compound of formula (Vi-B) : 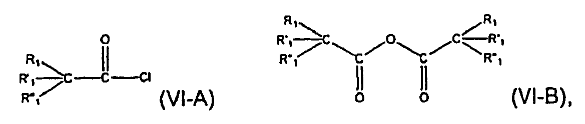 in which R₁, R'₁ and R"₁ have the same meaning as in claim 1, to obtain the following compound of formula (X) : 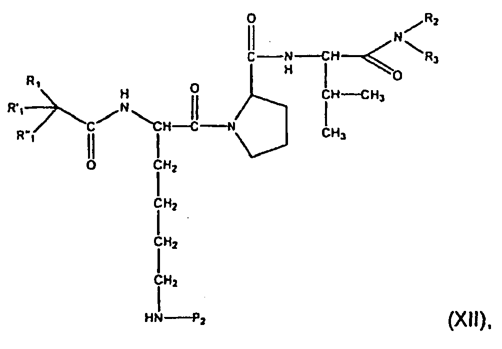 in which R₁, R'₁, R"₁, P₁ have the same meanings as in claim 1 and P₃ is a protective group;
b3) withdrawal of protective group P₃ from the compound of formula (X) to obtain the compound of formula (XI): 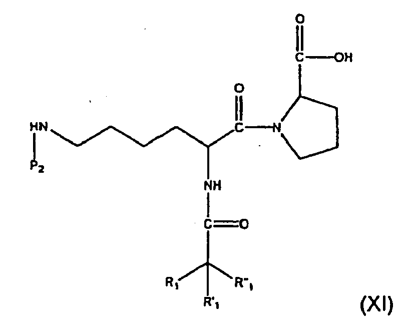 in which : R₁, R'₁, R"₁ and P₂ have the same meanings as in claim 1;
b4) coupling the compound of formula (XI) with the valine compound having the following formula (V), possibly in the form of a mineral or organic acid salt: 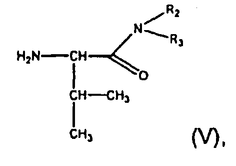 In which R₂ and R₃ have the same meaning as above, to obtain the following compound having formula (XII) : 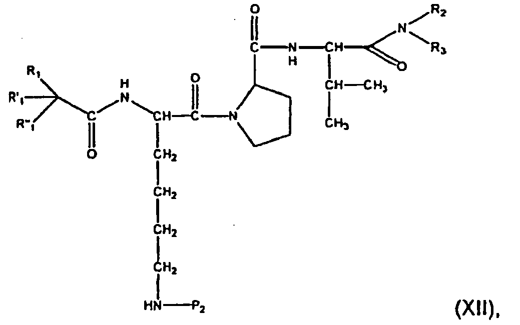 in which P₂, R₁, R'₁, R"₁, R₂ and R₃ have the same meaning as above.

13. The process according to anyone of claims 1 to 11, **characterized in that** step b) comprises the following steps:
b5) withdrawal of group P₃ from compound of formula (IV) in which group P₃ is a protective group, to obtain the compound having the following formula (VII): 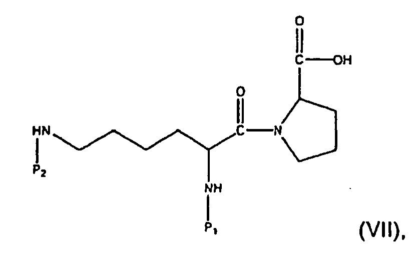 In which P₁ and P₂ have the same meaning as in claim 2;
b6) coupling the compounds of formula (VII) with the valine compound of formula (V), possibly in the form of a mineral or organic acid salt: 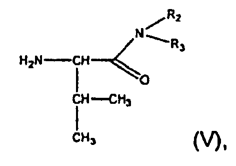 in which R₂ and R₃ have the same meaning as in claim 1 to obtain the compound of formula (XIII): 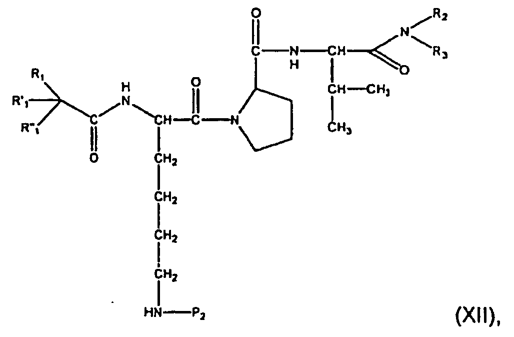 in which P₁, P₂, R₂ and R₃ have the same meaning as above;
b7) withdrawal of the protective group P₁ from compound of formula (XIII) to obtain the following compound of formula (XIV), possibly in the form of a mineral or organic acid salt: 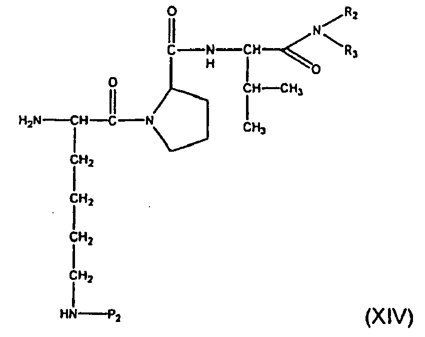 in which P₂, R₂ and R₃ have the same meaning as above;
b8) amidification of NH₂(α) group of the lysine moiety of the compound of formula (XIV) with the following compound of formula (VI-A) or the following compound of formula (VI-B), possibly in the form of a mineral or organic acid salt: 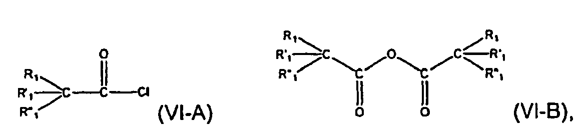 in which R₁, R'₁ and R"₁ have the same meaning as in claim 1, to obtain the following compound of formula (XII): 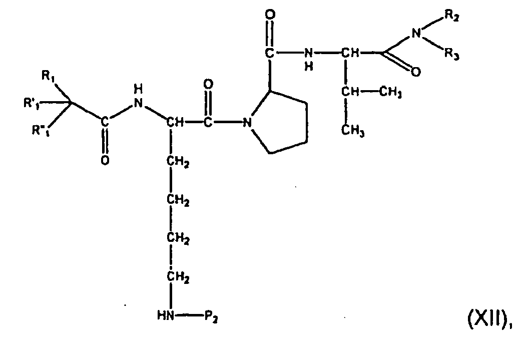 in which P₂, R₁, R'₁, R"₁, R₂ and R₃ have the same meaning as above.

14. The process according to anyone of claims 1 to 11, **characterized in that** step b) comprises the following steps:
b9) withdrawal of protective group P₁ from compound of formula (VII) in which group P₃ is a hydroxyl group to obtain the following compound of formula (XV): 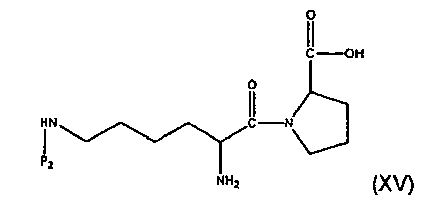 in which P₂ has the same meaning as in claim 2;
b10) amidification of NH₂(α) group of the lysine moiety of the compound of formula (XV) with the following compound of formula (VI-A) or the following compound of formula (VI-B): 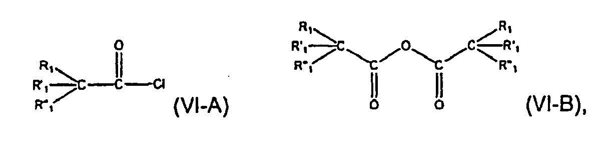 in which R₁, R'₁ and R"₁ have the same meaning as in claim 1, to obtain the following compound of formula (XI), possibly in the form of an organic or mineral base salt: 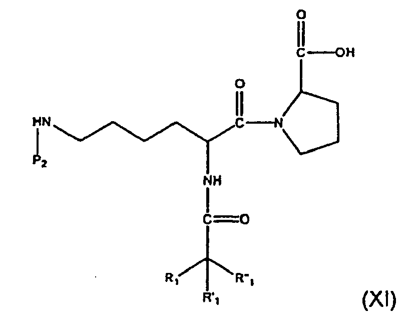 in which P₂, R₁, R'₁ and R"₁ have the same meaning as above;
b11) coupling the compound of formula (XI) with the valine compound of formula (V), possibly in the form of a mineral or organic acid salt: 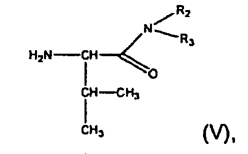 in which R₂ and R₃ have the same meaning as in claim 1; to obtain the compound of formula (XII): 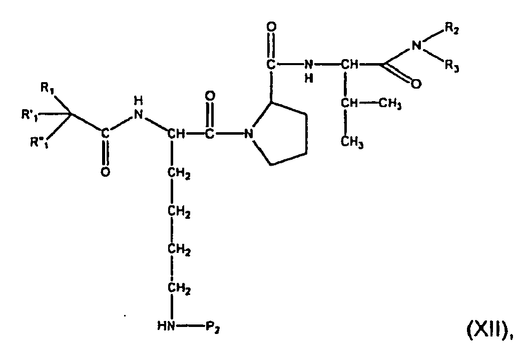 in which P₂, R₁, R'₁, R"₁, R₂ and R₃ have the same meaning as above.

15. The process according to anyone of claims 1 to 14, **characterized in that** in the compound of formula (II), the protective group P₁ is t-butyloxycarbonyl (BOC) and the protective group P₂ is benzyloxycarbonyl (Z).

16. The process according to anyone of claims 1 to 15, **characterized in that** in the compound of formula (III), the protective group P₃ is benzylic ester Obzl.

17. The process according to anyone of claims 1 to 16, **characterized in that** in the compound of formula (I), the groups R₁, R'₁ and R"₁ are each a hydrogen atom.

18. The process according to anyone of claims 1 to 14, **characterized in that** in the compound of formula (I), the groups R₂ and R₃ are each a hydrogen atom.

19. The process according to anyone of claims 1 to 14, **characterized in that** the protective group P₁ is t-butyloxyca0rbonyl (BOC), the protective group P₂ is benzyloxycarbonyl (Z) and the protective group P₃ is benzylic ester (Obzl).

20. Diamido derivative or diamido derivative salt of the KPV tripeptide having the following formula (IA) : 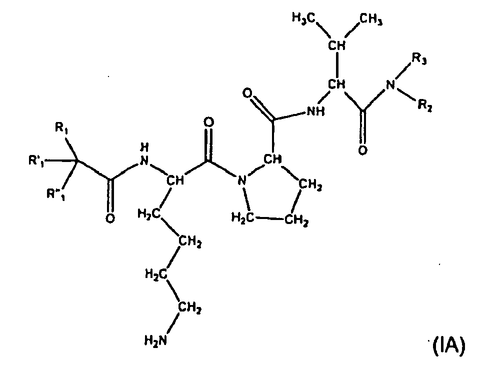 in which :
a) R₁, R'₁ and R"₁ are, independently from each other,
- a linear or branched C₁-C₂₂ alkyl, possibly interrupted by a heteroatom such as O or N or S or Si,
- a C₄-C₁₀ cycloalkyl,
- a linear or branched C₁-C₂₂ polyfluoroalkyl or perfluoroalkyl,
- an aryl possibly substituted by one or more halogen atom such as Cl, F, Br or I or, one or more linear or branched C₁-C₄ alkyl groups,
- an aralkyl
- or R₁ and R'₁ may form with C(R"₁) a saturated cycle of 3 to 7 atoms, possibly substituted by one or more linear or branched C₁-C₄ alkyl radicals and/or possibly including a heteroatom such as O, S or N,
- hydrogen
with the proviso that the group R₁(R'₁)(R"₁)CO is not an amido acid moiety or a peptidic moiety and at least one of R₁, R"₁ being other than hydrogen;
b) R₂ and R₃ are, independently from each other, a hydrogen atom or
- a linear or branched C₁-C₂₄ alkyl, possibly interrupted by a heteroatom such as O or N or S or Si,
- a C₄-C₁₀ cycloalkyl
- a linear or branched C₁-C₂₂ polyfluoroalkyl or perfluoroalkyl,
- an aryl, possibly substituted by one or more halogen atoms such as Cl, F, Br or I or, one or more linear or branched C₁-C₄ alkyl groups,
- an aralkyl,
- or R₂ and R₃ may form with the nitrogen atom a saturated cycle having 5 to 6 atoms, possibly substituted by one or more linear or branched C₁₋C₄ alkyl radicals, saturated cycle including optionally a heteroatom such as O, S or still an additional nitrogen atom,
at least one of R₂ or R₃ being other than hydrogen, and with the proviso that the N(R₂) (R₃) group is not an aminoacid or a peptidic moiety.

21. Diamido derivative salt of KPV tripeptide according to claim 20, **characterized in that** the salt is selected from chlorhydrates, bromhydrates, sulphates, acetates, citrates, tartrates, lactates; phosphates, hydrogenophosphates, propionates and succinates.

22. Diamido derivative or diamido derivative salt of KPV tripeptide according to claim 20 or 21, **characterized in that** the Lysine, Proline or Valine amino acids moieties can be anyone of the stereoisomers of each of these moieties.

23. Compositions containing in a physiologically acceptable medium a derivative (IA) according to anyone of claims 20 to 22.

24. Compositions according to claim 23, **characterized in that** the medium is a cosmetic medium and the derivative (IA) is present in an amount of 10⁻⁸ to 10⁻³ g/100g.

25. Composition according to claim 23 **characterized in that** the medium is a pharmaceutical medium and the derivative (IA) is present in an amount above 5.10⁻⁴ g/100g.

26. The use of the derivative (IA) according to anyone of claims 20 to 22 in a cosmetic composition or for the fabrication of a dermatologic composition for/or intended for treating dry skins and/or sensitive skins.

## Patentansprüche

1. Verfahren zur Synthese in Lösung eines Diamidderivats des Tripeptids KPV der folgenden Formel (I) oder eines Salzes der Verbindung der Formel (I): 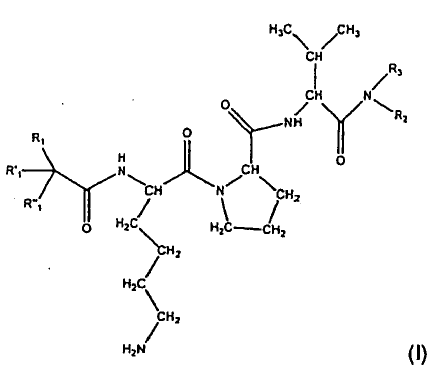 unabhängig von der Stereochemie der verwendeten Aminosäure, wobei
a) R₁, R'₁ und R"₁ unabhängig voneinander ein Wasserstoffatom oder
- lineares oder verzweigtes C₁-C₂₂-Alkyl, das gegebenenfalls durch ein Heteroatom wie O oder N oder S oder Si unterbrochen ist,
- C₄-C₁₀-Cycloalkyl,
- lineares oder verzweigtes C₁-C₂₂-Polyfluoralkyl oder
- Perfluoralkyl,
- Aryl, gegebenenfalls substituiert mit einem oder mehreren Halogenatomen wie Cl, F, Br oder I oder einer oder mehreren linearen oder verzweigten C₁-C₄-Alkylgruppen,
- Aralkyl darstellen
- oder R₁ und R'₁ mit C(R"₁) einen gesättigten Ring mit 3 bis 7 Atomen, der gegebenenfalls mit einem oder mehreren linearen oder verzweigten C₁-C₄-Alkylresten substituiert ist und/oder gegebenenfalls ein Heteroatom wie O, S oder N umfasst, bilden können,
unter der Maßgabe, dass die Gruppe R₁(R'₁) (R"₁)CO keinen Aminosäurerest oder Peptidrest darstellt;
b) R₂ und R₃ unabhängig voneinander ein Wasserstoffatom darstellen oder
- lineares oder verzweigtes C₁-C₂₄-Alkyl, das gegebenenfalls durch ein Heteroatom wie O oder N oder S oder Si unterbrochen ist,
- C₉-C₁₀-Cycloalkyl,
- lineares oder verzweigtes C₁-C₂₂-Polyfluoralkyl oder
- Perfluoralkyl,
- Aryl, gegebenenfalls substituiert mit einem oder mehreren Halogenatomen wie Cl, F, Br oder I oder einer oder mehreren linearen oder verzweigten C₁-C₄-Alkylgruppen,
- Aralkyl darstellen,
- oder R₂ und R₃ mit dem Stickstoffatom einen gesättigten Ring mit 5 oder 6 Atomen, der gegebenenfalls mit einem oder mehreren linearen oder verzweigten C₁-C₄-Alkylresten substituiert ist, bilden können, wobei ein gesättigter Ring gegebenenfalls ein Heteroatom wie O, S oder auch ein zusätzliches Stickstoffatom umfasst,
mit der Maßgabe, dass die Gruppe N(R₂) (R₃) keine Aminosäure oder keinen Peptidrest darstellt;
wobei das Verfahren **dadurch gekennzeichnet ist, dass** es die folgenden Stufen umfasst:
a) Reaktion eines zweifach geschützten Lysinrestes der folgenden Formel (II): 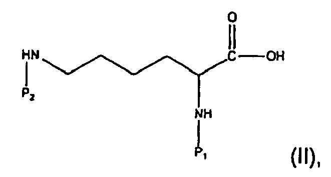 gegebenenfalls als Salz mit einer mineralischen oder organischen Base,
wobei P₁ und P₂, die voneinander verschieden sind, jeweils unabhängig voneinander eine Schutzgruppe darstellen,
mit einem Prolinrest der folgenden Formel (III): 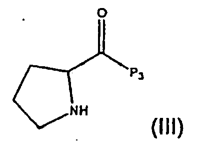 gegebenenfalls als Salz mit einer mineralischen oder organischen Säure,
wobei P₃ eine andere Schutzgruppe als eine der Schutzgruppen P₁ und P₂ darstellt oder wobei P₃ eine Hydroxylgruppe darstellt,
in Gegenwart eines Aktivierungsreagenzes oder eines Kupplungsreagenzes in einem Lösungsmittel unter Erhalt der Verbindung der folgenden Formel (IV): 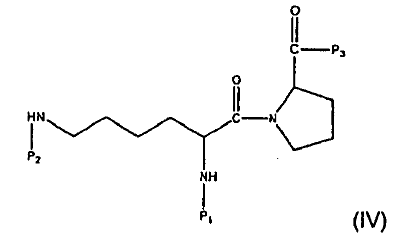 wobei P₁, P₂ und P₃, die unten angegebenen Bedeutungen haben;
b)
1) Kupplung an der C-terminalen Funktion des Prolinrestes der Verbindung der Formel (IV), in der P₃ für OH steht, einer Valinverbindung der folgenden Formel (V), gegebenenfalls als Salz mit einer mineralischen oder organischen Säure: 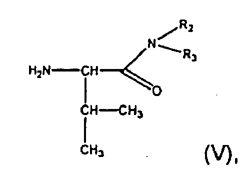 wobei R₂ und R₃ dieselbe Bedeutung wie oben haben, Eliminierung der Schutzgruppe P₁;
2) Amidierung der NH₂(α)-Gruppe in N-terminaler Position des Lysinrestes durch eine Verbindung der folgenden Formel (VI-A) oder (VI-B) 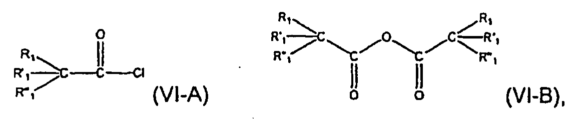 unter Erhalt der Verbindung der folgenden Formel (XII): 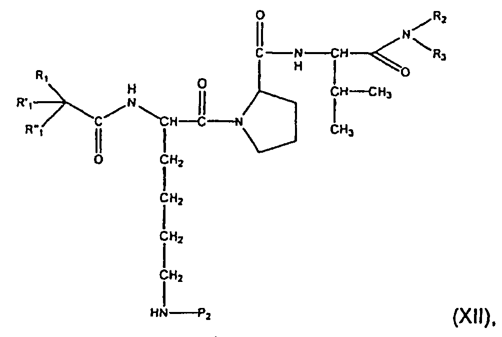 wobei P₂, R₁, R'₁, R''₁, R₂ und R₃ dieselbe Bedeutung wie oben haben;
wobei die Reihenfolge der Stufen 1) und 2) beliebig ist;
c) Eliminieren der Schutzgruppe P₂ der Verbindung der Formel (XII) unter Erhalt der Verbindung der Formel (I), gegebenenfalls in Form eines Mineralsalzes oder eines organischen Salzes.

2. Verfahren nach Anspruch 1, **dadurch gekennzeichnet, dass** das Salz aus den Chlorhydraten, Bromhydraten, Sulfaten, Acetaten, Citraten, Tartraten, Lactaten, Phosphaten, Hydrogenphosphaten, Propionaten und Succinaten ausgewählt wird.

3. Verfahren nach Anspruch 1 oder 2, **dadurch gekennzeichnet, dass** die Reste der Aminosäuren Lysin, Prolin oder Valin beliebige Stereoisomere jedes dieser Reste sein können.

4. Verfahren nach einem der Ansprüche 1 bis 3, **dadurch gekennzeichnet, dass** das Salz im Verlauf der Stufe c) durch Einführung der entsprechenden Säure erhalten wird.

5. Verfahren nach Anspruch 4, **dadurch gekennzeichnet, dass** die Säure Essigsäure oder Chlorwasserstoffsäure oder Bromwasserstoffsäure oder Schwefelsäure oder Citronensäure oder Weinsäure oder Milchsäure oder Phosphorsäure oder Hydrogenphosphorsäure oder Propionsäure oder Bernsteinsäure ist.

6. Verfahren nach Anspruch 5, **dadurch gekennzeichnet, dass** die Säure Essigsäure oder Chlorwasserstoffsäure ist.

7. Verfahren nach einem der vorangehenden Ansprüche, **dadurch gekennzeichnet, dass** die Schutzgruppen P₁ und P₂ unabhängig voneinander für Adoc (= 1-Adamantyloxycarbonyl), BOC (= t-Butyloxycarbonyl), 2-Brom-Z (= 2-Brombenzyloxycarbonyl) oder 2-Chlor-Z (= 2-Chlorbenzyloxycarbonyl) oder Fmoc (= 9-Fluorenylmethoxycarbonyl) oder Formyl oder Nicotinoyl oder 4-Nitro-Z (= 4-Nitrobenzyloxycarbonyl) oder Tfa (= Trifluoracetyl) oder Tos (= p-Toluolsulfonyl) oder Z (= Benzyloxycarbonyl) oder Adpoc (= 1-(Adamantyl)-1-methylethoxycarbonyl) stehen.

8. Verfahren nach Anspruch 7, **dadurch gekennzeichnet, dass** die Schutzgruppen P₁ und P₂ so gewählt sind, dass sie bei unterschiedlichen Arbeitsbedingungen eliminiert werden.

9. Verfahren nach einem der vorangehenden Ansprüche, **dadurch gekennzeichnet, dass** die Verbindung der Formel (II) als Salz mit einer organischen Base, vorzugsweise einem organischen Amin, vorliegt.

10. Verfahren nach einem der vorangehenden Ansprüche, **dadurch gekennzeichnet, dass** die Verbindung der Formel (III) als Salz mit einer Mineralsäure oder organischen Säure vorliegt.

11. Verfahren nach einem der vorangehenden Ansprüche, **dadurch gekennzeichnet, dass** in Stufe a) die Kupplungsreaktion in Gegenwart eines Kupplungsreagenzes oder Aktivierungsreagenzes, ausgewählt unter den Carbodiimiden wie zum Beispiel DCC (= Dicyclohexylcarbodiimid) oder den wasserlöslichen Formen von Carbodiimiden wie EDC (= N-Ethyl-N'-(3-dimethylaminopropyl)carbodiimidchlorhydrat), Phosphoniumsalzen wie BOP (= Benzotriazol-1-yloxy)-tris(dimethylamino)phosphoniumhexafluorphosphat), PyBOP (= (Benzotriazol-1-yloxy)-tripyrrolidinophosphoniumhexafluorphosphat), PyBROP (= Bromtripyrrolidinophosphoniumhexafluorphosphat), PyCloP (= Chlortripyrrolidinophosphoniumhexafluorphosphat), oder auch mit Hilfe von Reagenzien wie PyClU (= Chlor-N,N,N',N'-bis(tetramethylen)formamidiniumhexafluorphosphat), N-Hydroxysuccinimid, EEDQ (= 1-Ethoxycarbonyl-2-ethoxy-1,2-dihydrochinolin), CDI (= Carbonyldiimidazol) oder auch Chlorformiaten wie Ethylchlorformiat oder Isobutylchlorformiat, durchgeführt wird.

12. Verfahren nach einem der vorangehenden Ansprüche, **dadurch gekennzeichnet, dass** die Stufe b) die folgenden Stufen umfasst:
b1) Eliminierung der Schutzgruppe P₁ aus der Verbindung der Formel (IV), in der P₃ eine Schutzgruppe darstellt, derart, dass die Verbindung der Formel (IX) erhalten wird: 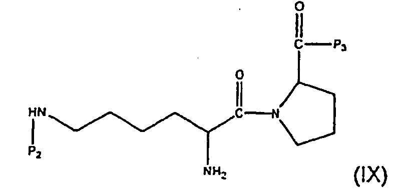 wobei P₁ dieselbe Bedeutung wie in Anspruch 1 hat und P₃ eine Schutzgruppe darstellt;
b2) Amidierung der Gruppe NH₂(α) des Lysinrestes der Verbindung der Formel (IX) mit der Verbindung der Formel (VI-A) oder der Verbindung der folgenden Formel (VI-B): 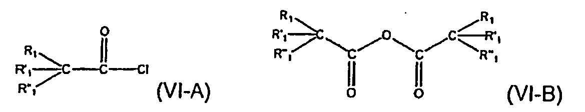 wobei R₁, R'₁ und R"₁ dieselben Bedeutungen wie in Anspruch 1 haben,
um die Verbindung der folgenden Formel (X) zu erhalten: 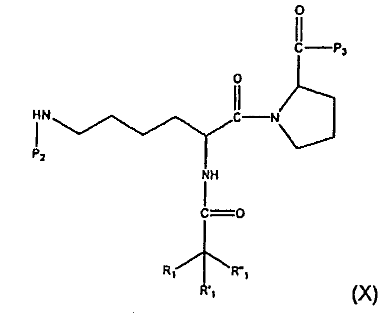 wobei R₁, R'₁, R''₁, P₁ dieselbe Bedeutung wie in Anspruch 1 haben und P₃ eine Schutzgruppe darstellt;
b3) Eliminierung der Schutzgruppe P₃ der Verbindung der Formel (X) unter Erhalt der Verbindung der Formel (XI): 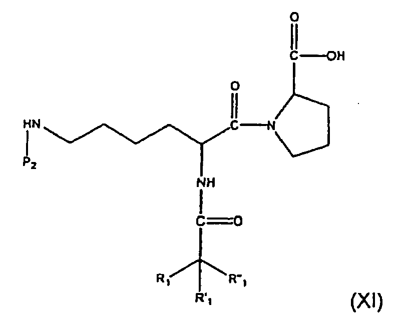 wobei : R₁, R'₁, R"₁ und P₂ dieselbe Bedeutung wie in Anspruch 1 haben;
b4) Kuppeln der Verbindung der Formel (XI) mit der Valinverbindung der folgenden Formel (V), gegebenenfalls als Salz mit einer Mineralsäure oder organischen Säure: 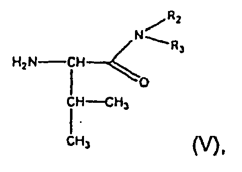 wobei R₂ und R₃ dieselbe Bedeutung wie oben haben, um die Verbindung der folgenden Formel (XII) zu erhalten: 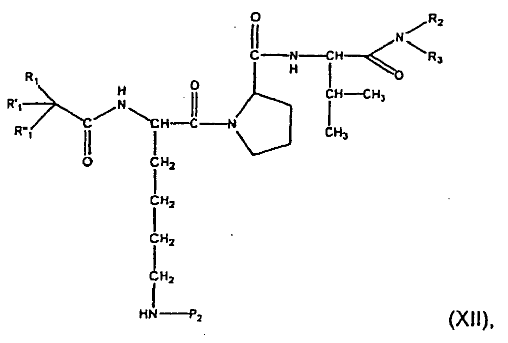 wobei P₂, R₁, R'₁, R''₁, R₂ und R₃ dieselbe Bedeutung wie oben haben.

13. Verfahren nach einem der Ansprüche 1 bis 11, **dadurch gekennzeichnet, dass** die Stufe b) die folgenden Stufen umfasst:
b5) Eliminieren der Gruppe P₃ der Verbindung der Formel (IV), in der die Gruppe P₃ eine Schutzgruppe darstellt, um die Verbindung der folgenden Formel (VII) zu erhalten: 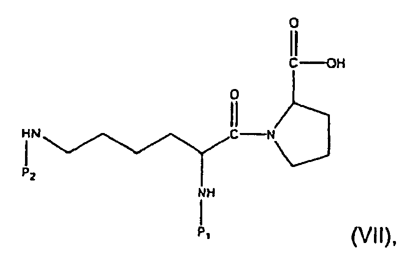 wobei P₁ und P₂ dieselbe Bedeutung wie in Anspruch 2 haben;
b6) Kupplung der Verbindung der Formel (VII) mit der Valinverbindung der Formel (V), gegebenenfalls als Salz mit einer mineralischen oder organischen Säure: 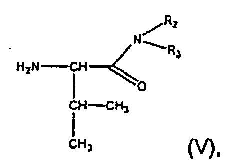 wobei R₂ und R₃ dieselbe Bedeutung wie in Anspruch 1 haben, um eine Verbindung der Formel (XIII) zu erhalten: 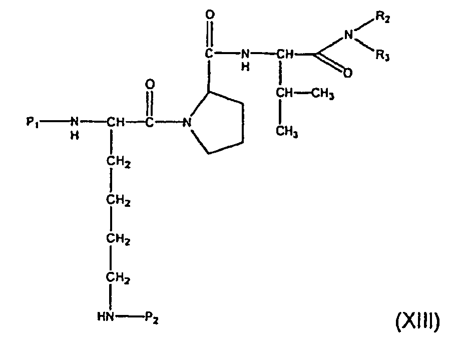 wobei P₁, P₂, R₂ und R₃ dieselbe Bedeutung wie oben haben;
b7) Eliminieren der Schutzgruppe P₁ aus der Verbindung der Formel (XIII), um die Verbindung der folgenden Formel (XIV), gegebenenfalls als Salz mit einer mineralischen Säure oder organischen Säure, zu erhalten: 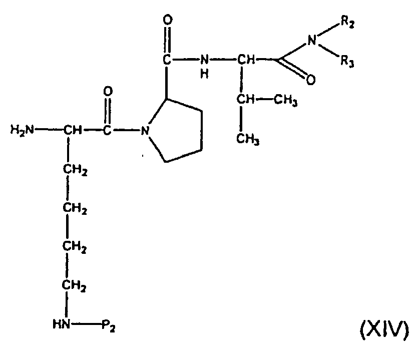 wobei P₂, R₂ und R₃ dieselbe Bedeutung wie oben haben;
b8) Amidierung der NH₂(α)-Gruppe des Lysinrests der Verbindung der Formel (XIV) mit der Verbindung der Formel (VI-A) oder der Verbindung der folgenden Formel (VI-B), gegebenenfalls als Salz mit einer Mineralsäure oder organischen Säure: 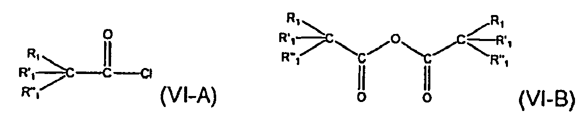 wobei R₁, R'₁ und R"₁ dieselben Bedeutungen wie in Anspruch 1 haben,
um die Verbindung der folgenden Formel (XII) zu erhalten: 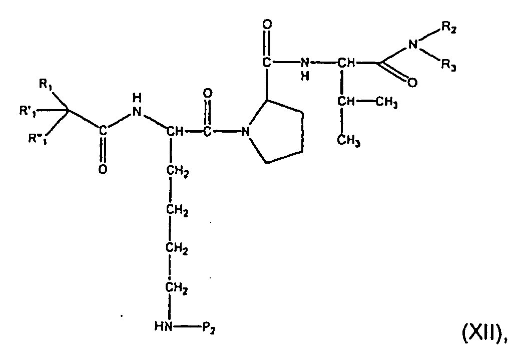 wobei P₂, R₁, R'₁, R"₁, R₂ und R₃ dieselbe Bedeutung wie oben haben.

14. Verfahren nach einem der Ansprüche 1 bis 11, **dadurch gekennzeichnet, dass** die Stufe b) die folgenden Stufen umfasst:
b9) Eliminieren der Schutzgruppe P₁ der Verbindung der Formel (VII), in der die Gruppe P₃ eine Hydroxylgruppe darstellt, um die Verbindung der folgenden Formel (XV) zu erhalten: 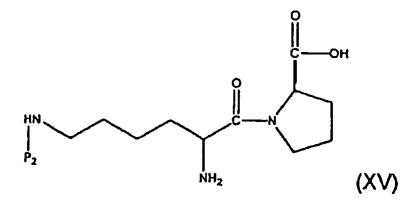 wobei P₂ dieselbe Bedeutung wie in Anspruch 2 hat;
b10) Amidbildung der NH₂(α)-Gruppe des Lysinrestes der Verbindung der Formel (XV) mit der Verbindung der Formel (VI-A) oder der Verbindung der folgenden Formel (VI-B): 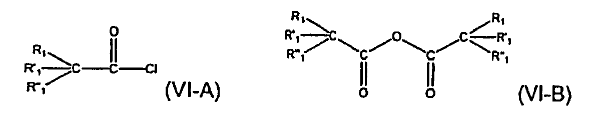 wobei R₁, R'₁ und R"₁ dieselben Bedeutungen wie in Anspruch 1 haben,
um die Verbindung der folgenden Formel (XI), gegebenenfalls als Salz mit einer organischen Base oder einer mineralischen Base, zu erhalten: 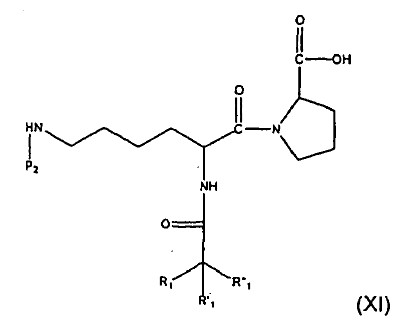 wobei P₂, R₁, R'₁ und R"₁ dieselbe Bedeutung wie oben haben;
b11) Kupplung der Verbindung der Formel (XI) mit der Valinverbindung der folgenden Formel (V), gegebenenfalls als Salz mit einer Mineralsäure oder organischen Säure: 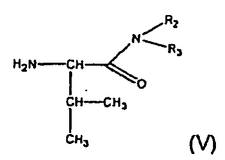 wobei R₂ und R₃ dieselbe Bedeutung wie in Anspruch 1 haben;
um die Verbindung der Formel (XII) zu erhalten: 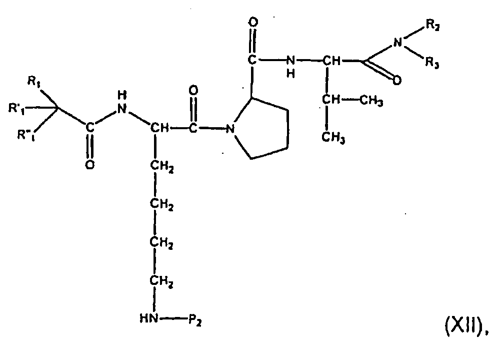 wobei P₂, R_{1'} R'₁, R"₁, R₂ und R₃ dieselbe Bedeutung wie oben haben.

15. Verfahren nach einem der Ansprüche 1 bis 14, **dadurch gekennzeichnet, dass** in der Verbindung der Formel (II) die Schutzgruppe P₁ t-Butyloxycarbonyl (BOC) ist und die Schutzgruppe P₂ Benzyloxycarbonyl (Z) ist.

16. Verfahren nach einem der Ansprüche 1 bis 15, **dadurch gekennzeichnet, dass** in der Verbindung der Formel (III) die Schutzgruppe P₃ die Benzylestergruppe OBzl ist.

17. Verfahren nach einem der Ansprüche 1 bis 16, **dadurch gekennzeichnet, dass** in der Verbindung der Formel (I) die Gruppen R₁, R'₁ und R"₁ jeweils ein Wasserstoffatom darstellen.

18. Verfahren nach einem der Ansprüche 1 bis 14, **dadurch gekennzeichnet, dass** in der Verbindung der Formel (I) die Gruppen R₂ und R₃ jeweils ein Wasserstoffatom darstellen.

19. Verfahren nach einem der Ansprüche 1 bis 14, **dadurch gekennzeichnet, dass** die Schutzgruppe P₁ t-Butyloxycarbonyl (BOC) ist, die Schutzgruppe P₂ Benzyloxycarbonyl (Z) ist und die Schutzgruppe P₃ der Benzylester OBzl ist.

20. Diamidderivat oder Salz des Diamidderivats des Tripeptids KPV der folgenden Formel (IA): 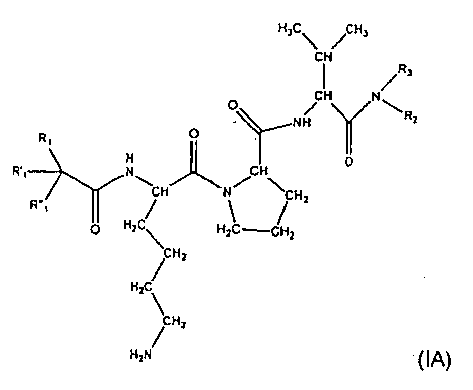 wobei:
a) R₁, R'₁ und R"₁ unabhängig voneinander darstellen:
- lineares oder verzweigtes C₁-C₂₂-Alkyl, gegebenenfalls unterbrochen durch ein Heteroatom wie O oder N oder S oder Si,
- C₄-C₁₀-Cycloalkyl,
- lineares oder verzweigtes C₁-C₂₂-Polyfluoralkyl oder
- Perfluoralkyl,
- Aryl, gegebenenfalls substituiert mit einem oder mehreren Halogenatomen wie Cl, F, Br oder I oder einer oder mehreren linearen oder verzweigten C₁-C₄-Alkylgruppen,
- Aralkyl
- oder R₁ und R'₁ mit C(R"₁) einen gesättigten Ring mit 3 bis 7 Atomen, der gegebenenfalls mit einem oder mehreren linearen oder verzweigten C₁-C₄-Alkylresten substituiert ist und/oder gegebenenfalls ein Heteroatom wie O, S oder N umfasst, bilden können,
- Wasserstoff,
unter der Maßgabe, dass die Gruppe R₁(R'₁) (R"₁)CO keinen Aminosäurerest oder Peptidrest darstellt, wobei wenigstens eines der R₁, R'₁, R" ₁ von Wasserstoff verschieden ist;
b) R₂ und R₃ unabhängig voneinander ein Wasserstoffatom darstellen oder
- lineares oder verzweigtes C₁-C₂₄-Alkyl, das gegebenenfalls durch ein Heteroatom wie O oder N oder S oder Si unterbrochen ist,
- C₄-C₁₀-Cycloalkyl,
- lineares oder verzweigtes C₁-C₂₂-Polyfluoralkyl oder
- Perfluoralkyl,
- Aryl, gegebenenfalls substituiert mit einem oder mehreren Halogenatomen wie Cl, F, Br oder I oder einer oder mehreren linearen oder verzweigten C₁-C₄-Alkylgruppen,
- Aralkyl darstellen,
- oder R₂ und R₃ mit dem Stickstoffatom einen gesättigten Ring mit 5 oder 6 Atomen, der gegebenenfalls mit einem oder mehreren linearen oder verzweigten C₁-C₄-Alkylresten substituiert ist, bilden können, wobei ein gesättigter Ring gegebenenfalls ein Heteroatom wie O, S oder auch ein zusätzliches Stickstoffatom umfasst,
wobei wenigstens einer der Reste R₂ oder R₃ von Wasserstoff verschieden ist, mit der Maßgabe, dass die Gruppe N(R₂)(R₃) keinen Aminosäurerest oder Peptidrest darstellt.

21. Salz eines Diamidderivats des Tripeptids KPV gemäß Anspruch 20, **dadurch gekennzeichnet, dass** das Salz unter den Chlorhydraten, Bromhydraten, Sulfaten, Acetaten, Citraten, Tartraten, Lactaten, Phosphaten, Hydrogenphosphaten, Propionaten und Succinaten ausgewählt ist.

22. Diamidderivat oder Salz des Diamidderivats des Tripeptids KPV gemäß Anspruch 20 oder 21, **dadurch gekennzeichnet, dass** die Reste der Aminosäuren Lysin, Prolin oder Valin ein beliebiges der Stereoisomeren jeder dieser Reste sein können.

23. Zusammensetzungen, die in einem physiologisch annehmbaren Medium ein Derivat (IA) nach einem der Ansprüche 20 bis 22 enthalten.

24. Zusammensetzungen nach Anspruch 23, **dadurch gekennzeichnet, dass** das Milieu ein kosmetisches Milieu ist und das Derivat (IA) mit einem Gehalt von 10⁻⁸ bis 10⁻³ g/100 g vorliegt.

25. Zusammensetzung nach Anspruch 23, **dadurch gekennzeichnet, dass** das Milieu ein pharmazeutisches Milieu ist und dass das Derivat (IA) in einem Gehalt von über 5·10⁻⁴ g/100 g vorliegt.

26. Verwendung eines Derivats (IA) nach einem der Ansprüche 20 bis 22 in einer kosmetischen Zusammensetzung oder zur Herstellung einer dermatologischen Zusammensetzung für/oder bestimmt zur Behandlung von trockener Haut und/oder empfindlicher Haut.
